# EUROPÄISCHE PATENTSCHRIFT

(11) **EP 0 581 730 B1**
(45) Veröffentlichungstag und Bekanntmachung des Hinweises auf die Patenterteilung: **20.01.1999**
(21) Anmeldenummer: 93810504.6
(22) Anmeldetag: 14.07.1993
(51) Int. Cl.: C09B 62/447, C09B 62/026

(54) **Reaktivfarbstoffe, Verfahren zu deren Herstellung und deren Verwendung**
Reactive dyes, process for their manufacture and their use
Colorants réactifs, procédé pour leur préparation et leur utilisation

(30) Priorität: 23.07.1992 CH 2317/92
(43) Veröffentlichungstag der Anmeldung: 02.02.1994
(73) Patentinhaber: Ciba Specialty Chemicals Holding Inc., 4057 Basel (CH)
(72) Erfinder: Herzig, Paul, CH-4057 Basel (CH); Tzikas, Athanassios, Dr., CH-4133 Pratteln (CH); Andreoli, Anton, CH-4125 Riehen (CH); Carisch, Claudia, CH-4153 Reinach (CH)

(56) Entgegenhaltungen:
- DE-A- 3 227 253

## Beschreibung

Die vorliegende Erfindung betrifft neue Reaktivfarbstoffe, Verfahren zu deren Herstellung und deren Verwendung zum Färben oder Bedrucken von Fasermaterialien.

Die Praxis des Färbens mit Reaktivfarbstoffen hat in neuerer Zeit zu erhöhten Anforderungen an die Qualität der Färbungen und die Wirtschaftlichkeit des Färbeprozesses geführt. Infolge dessen besteht weiterhin ein Bedarf nach neuen Reaktivfarbstoffen, welche verbesserte Eigenschaften, insbesondere in bezug auf die Applikation, aufweisen.

Für das Färben werden heute Reaktivfarbstoffe gefordert, die eine ausreichende Substantivität haben und die zugleich eine gute Auswaschbarkeit der nicht fixierten Anteile aufweisen. Sie sollen ferner eine gute färberische Ausbeute aufweisen und hohe Reaktivität besitzen, wobei insbesondere Färbungen mit hohen Fixiergraden geliefert werden sollen. Von den bekannten Farbstoffen werden diese Anforderungen nicht in allen Eigenschaften erfüllt.

Der vorliegenden Erfindung liegt daher die Aufgabe zugrunde, neue, verbesserte Reaktivfarbstoffe für das Färben und Bedrucken von Fasermaterialien zu finden, welche die oben charakterisierten Qualitäten in hohem Masse besitzen. Die neuen Farbstoffe sollten sich vor allem durch hohe Fixierausbeuten und hohe Faser-Farbstoff-Bindungsstabilitäten auszeichnen, und ausserdem sollten die nicht auf der Faser fixierten Anteile leicht auswaschbar sein. Sie sollten ferner Färbungen mit guten Allgemeinechtheiten, beispielsweise Licht- und Nassechtheiten, ergeben.

Es hat sich gezeigt, dass mit den weiter unten definierten neuen reaktiven Farbstoffen die gestellte Aufgabe weitgehend gelöst wird.

Gegenstand der Erfindung sind daher Reaktivfarbstoffe der Formel 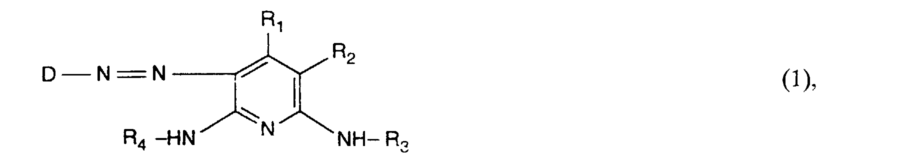 worin D der Rest einer Diazokomponente der Benzol- oder Naphthalinreihe oder der Rest eines Mono- oder Disazofarbstoffes ist,
R₁ C₁-C₄-Alkyl,
R₂ Cyano, Carbamoyl oder Sulfomethyl ist und
R₃ und R₄ unabhängig voneinander Wasserstoff, gegebenenfalls durch Hydroxyl, Sulfo oder Sulfato substituiertes und mit Ausnahme von Methyl gegebenenfalls durch Sauerstoff unterbrochenes C₁-C₁₂-Alkyl,
oder einen Rest der Formel 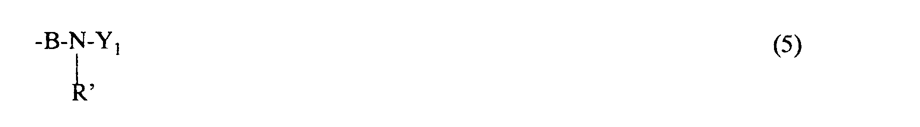 bedeuten, worin
R' Wasserstoff oder unsubstituiertes oder durch Hydroxyl, Sulfo oder Sulfato substituiertes und mit Ausnahme von Methyl gegebenenfalls durch Sauerstoff unterbrochenes C₁-C₁₂-Alkyl ist oder der Rest -(NR')- einen bivalenten 5- bis 7-gliedrigen aliphatischen Heterocyclus darstellt,
B gegebenenfalls durch Hydroxyl, Sulfo oder Sulfato substituiertes, und gegebenenfalls durch Sauerstoff unterbrochenes C₂-C₁₂-Alkylen und
Y₁ ein faserreaktiver Rest der Halogentriazin- oder Halogenpyrimidinreihe ist, und der Reaktivfarbstoff der Formel (1) als faserreaktiven Rest einen Rest der Halogentriazinreihe, der Halogenpyrimidinreihe oder einen Rest der Formel

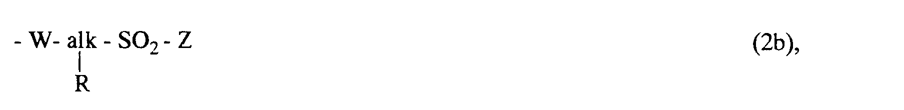

-W-alk-E-alk'-SO₂-Z (2c),

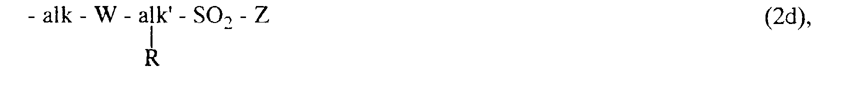

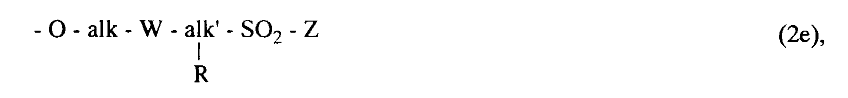

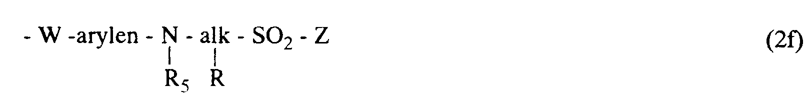

oder

-NH-CO-Z₁ (2g),

worin
W eine Gruppe der Formel -SO₂-NR₅-, -CONR₅- oder -NR₅CO- ist,
R₅ Wasserstoff, unsubstituiertes oder durch Hydroxy Sulfo, Sulfato, Carboxy oder Cyano substituiertes C₁-C₄-Alkyl,
R Wasserstoff, Hydroxy, Sulfo, Sulfato, Carboxy, Cyano, Halogen, C₁-C₄-Alkoxycarbonyl, C₁-C₄-Alkanoyloxy oder Carbamoyl ist,
Z eine Gruppe der Formel -CH=CH₂ oder -CH₂-CH₂-U₁ und U₁ eine Abgangsgruppe ist,
Z₁ eine Gruppe der Formel 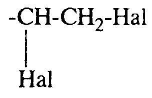 oder 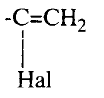 und Hal Halogen bedeutet,
E der Rest -O- oder -NR₆- und R₆ Wasserstoff oder C₁-C₄-Alkyl ist,
alk und alk' unabhängig voneinander C₁-C₆-Alkylen bedeuten und
arylen einen unsubstituierten oder durch Sulfo, Carboxy, C₁-C₄-Alkyl, C₁-C₄-Alkoxy oder Halogen substituierten Phenylen- oder Naphthylenrest bedeutet, enthält,
wobei der Reaktivfarbstoff der Formel (1) mindestens eine permanente Sulfo oder Sulfatogruppe und nur einen faserreaktiven Rest enthält.

Aus der DE-A-32 27 253 sind Farbstoffe bekannt, welche sich von den erfindungsgemässen Farbstoffen hinsichtlich eines der Reste R₃ und R₄ unterscheiden und zudem keine permanente Sulfo oder Sulfatogruppe enthalten.

Als Beispiele für Substituenten im Rest D seien genannt: Alkylgruppen mit 1 bis 12 Kohlenstoffatomen, insbesondere 1 bis 4 Kohlenstoffatomen, wie Methyl, Aethyl, Propyl, Isopropyl oder Butyl, Alkoxygruppen mit 1 bis 8 Kohlenstoffatomen, insbesondere 1 bis 4 Kohlenstoffatomen, wie Methoxy, Aethoxy, Propoxy, Isopropoxy oder Butoxy, Alkanoylaminogruppen mit 2 bis 8 Kohlenstoffatomen, insbesondere 2 bis 4 Kohlenstoffatomen, wie Acetylamino oder Propionylamino, Phenyl- oder Naphthylamino, Alkoxycarbonylaminogruppen mit 2 bis 8 Kohlenstoffatomen, insbesondere 2 bis 4 Kohlenstoffatomen, Alkanoylgruppen mit 2 bis 8 Kohlenstoffatomen, insbesondere 2 bis 4 Kohlenstoffatomen, Alkoxycarbonyl mit 1 bis 4 Kohlenstoffatomen im Alkoxyrest, wie Methoxycarbonyl oder Aethoxycarbonyl, Alkylsulfonyl mit 1 bis 4 Kohlenstoffatomen, wie Methylsulfonyl oder Aethylsulfonyl, Phenyl- oder Naphthylsulfonyl, Alkoxycarbonyl mit 1 bis 4 Kohlenstoffatomen im Alkoxyrest, wie Methoxycarbonyl oder Aethoxycarbonyl, Benzoyl, gegebenenfalls am Stickstoff durch C₁-C₄-Alkyl substituiertes Benzoylamino, Phenyl, Naphthyl, Amino, durch C₁-C₁₂-Alkyl, Phenyl oder Naphthyl mono- oder disubstituiertes Amino, Trifluormethyl, Nitro, Cyano, Hydroxyl, Halogen, wie Fluor, Chlor oder Brom, Carbamoyl, N-Alkylcarbamoyl mit 1 bis 4 Kohlenstoffatomen im Alkylrest, wie N-Methylcarbamoyl oder N-Aethylcarbamoyl, Sulfamoyl, N-Alkylsulfamoyl mit 1 bis 4 Kohlenstoffatomen, wie N-Methylsulfamoyl, N-Aethylsulfamoyl, N-Propylsulfamoyl, N-Isopropylsulfamoyl oder N-Butylsulfamoyl, N-(β-Hydroxyäthyl)-sulfamoyl, N,N-Di-(β-hydroxyäthyl)-sulfamoyl, N-Phenylsulfamoyl, Ureido, Carboxy, Sulfomethyl, Sulfo oder Sulfato sowie faserreaktive Reste, wobei die einen Alkyl-, Phenyl- oder Naphthylrest enthaltenden Substituenten im Alkyl-, Phenyl- oder Naphthylrest weitersubstituiert sein können, wie z.B. durch die oben für D genannten Substituenten. Die Alkylreste können zudem durch Sauerstoff (-O-) unterbrochen sein.

Unter faserreaktiven Resten sind solche zu verstehen, die mit den Hydroxygruppen der Cellulose, den Amino-, Carboxy-, Hydroxy- und Thiolgruppen bei Wolle und Seide, oder mit den Amino- und eventuell Carboxygruppen von synthetischen Polyamiden unter Bildung kovalenter chemischer Bindungen zu reagieren vermögen. Die faserreaktiven Reste sind in der Regel direkt oder über ein Brückenglied an den Farbstoffrest gebunden. Geeignete faserreaktive Reste sind beispielsweise solche, die mindestens einen abspaltbaren Substituenten an einem aliphatischen, aromatischen oder heterocyclischen Rest enthalten oder worin die genannten Reste einen zur Reaktion mit dem Fasermaterial geeigneten Rest, wie z.B. einen Vinylrest, enthalten.

Unter permanenten Sulfo- oder Sulfatogruppen sind solche zu verstehen, welche bei der Reaktion des Reaktivfarbstoffes mit dem Fasermaterial nicht abgespalten werden. Vorzugsweise enthalten die Reaktivfarbstoffe der Formel (1) 1 bis 5, insbesondere 1 bis 3 permanente Sulfo- oder Sulfatogruppen.

Die Reaktivfarbstoffe der Formel (1) enthalten nur einen faserreaktiven Rest, welcher entweder am Rest D, am Rest R₃ oder am Rest R₄ gebunden ist. Als Reaktivreste der Halogentriazinreihe kommen insbesondere solche der Formel 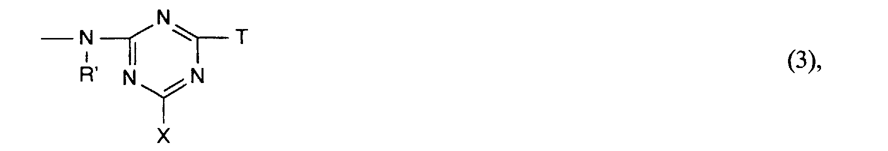 worin R' Wasserstoff oder gegebenenfalls substituiertes und mit Ausnahme von Methyl gegebenenfalls durch Sauerstoff unterbrochenes C₁-C₁₂-Alkyl bedeutet und der Rest -(NR')- einen bivalenten 5- bis 7-gliedrigen aliphatischen Heterocyclus darstellen kann, X eine als Anion abspaltbare Gruppe und T eine als Anion abspaltbare Gruppe oder ein nicht-reaktiver Rest ist, in Betracht.

Als Reaktivreste der Halogenpyrimidinreihe kommen insbesondere solche der Formel 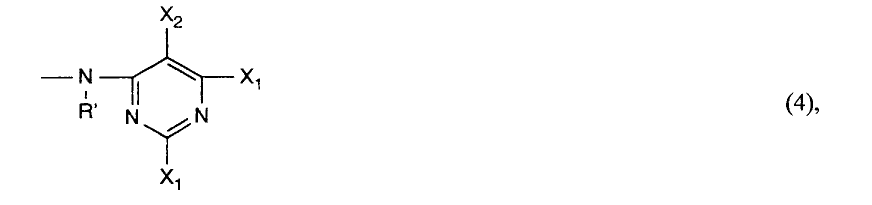 worin R' die oben angegebenen Bedeutungen hat, einer der Reste X₁ eine als Anion abspaltbare Gruppe ist, der andere Rest X₁ eine als Anion abspaltbare Gruppe oder ein nicht-reaktiver Substituent ist und X₂ ein negativer Substituent ist, in Betracht.

Geeignete Abgangsgruppen U₁ sind z.B. -Cl, -Br, -F, -OSO₃H, -SSO₃H, -OCO-CH₃, -OPO₃H₂, -OCO-CCl₃, -OCO-CHCl₂, -OCO-CH₂Cl, -OSO₂-C₁-C₄-Alkyl, -OSO₂-N(C₁-C₄-Alkyl)₂ oder -OCO-C₆H₅.

Bevorzugt ist U₁ eine Gruppe der Formel -Cl, -OSO₃H, -SSO₃H, -OCO-CH₃, -OCO-C₆H₅ oder -OPO₃H₂, insbesondere -Cl oder -OSO₃H, vorzugsweise -OSO₃H.

Bei alk und alk' handelt es sich unabhängig voneinander z.B. um einen Methylen-, Äthylen-, 1,3-Propylen-, 1,4-Butylen-, 1,5-Pentylen- oder 1,6-Hexylenrest oder deren verzweigte Isomere.

Bevorzugt stehen alk und alk' für einen C₁-C₄-Alkylenrest und insbesondere bevorzugt für einen Äthylenrest.

Arylen ist vorzugsweise ein 1,3- oder 1,4-Phenylenrest, der unsubstituiert oder z.B. durch Sulfo, Methyl, Methoxy oder Carboxy substituiert ist.

R bedeutet bevorzugt Wasserstoff.

R₅ ist vorzugsweise Wasserstoff oder C₁-C₄-Alkyl, wie Methyl, Aethyl, Propyl, Isopropyl, Butyl, Isobutyl, sek.-Butyl oder tert.-Butyl. Besonders bevorzugt ist R₅ Wasserstoff.

E steht vorzugsweise für -NH- oder -O- und insbesondere bevorzugt für -O-.

W bedeutet bevorzugt eine Gruppe der Formel -CONH- oder -NHCO-, insbesondere eine Gruppe der Formel -CONH-.

Hal im Rest Z₁ ist vorzugsweise Chlor oder insbesondere Brom.

Bevorzugt als Reaktivreste der Formeln (2b) bis (2g) sind solche, worin W eine Gruppe der Formel -CONH- oder -NHCO-, R und R₅ Wasserstoff, E der Rest -O- oder -NH-, Hal Chlor oder Brom und U₁ eine Gruppe der Formel -Cl, -OSO₃H, -SSO₃H, -OCO-CH₃, -OCO-C₆H₅ oder -OPO₃H₂, insbesondere eine Gruppe der Formel -Cl oder -OSO₃H, ist.

R' ist vorzugsweise Wasserstoff oder unsubstituiertes oder durch Hydroxyl, Sulfo oder Sulfato substituiertes und mit Ausnahme von Methyl gegebenenfalls durch Sauerstoff unterbrochenes C₁-C₁₂-Alkyl, oder der Rest -(NR')- stellt einen bivalenten 5- bis 7-gliedrigen aliphatischen Heterocyclus dar.

Als 5- bis 7-gliedriger aliphatischer Heterocyclus kommt für den Rest -(NR')-insbesondere der Rest der Formel 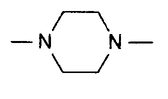 in Betracht.

Der Rest R' kann durch Sauerstoff unterbrochen sein, wie z.B. durch 1 bis 3, insbesondere 1 oder 2 Reste -O-.

Bevorzugt ist R' Wasserstoff oder unsubstituiertes oder durch Hydroxyl, Sulfo oder Sulfato substituiertes und mit Ausnahme von Methyl gegebenenfalls durch 1 bis 3 Reste -O- unterbrochenes C₁-C₁₂-Alkyl, insbesondere C₁-C₈-Alkyl, oder der Rest -(NR')- stellt einen Rest der Formel 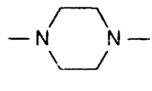 dar.

Beispiele für R' sind Methyl, Aethyl, Propyl, Isopropyl, Butyl, Isobutyl, sek.-Butyl, tert.-Butyl oder geradkettiges oder verzweigtes Pentyl, Hexyl oder Octyl, 2-Hydroxyäthyl, 3-Hydroxypropyl, 2-Sulfatoäthyl, 3-Sulfatopropyl, 2-Sulfoäthyl, 3-Sulfopropyl, 2-Methoxyäthyl, 3-Methoxypropyl, sowie Reste der Formeln -(CH₂)₂-O-(CH₂)₂-OH und -(CH₂)₂-O-(CH₂)₂-OSO₃H.

Ganz besonders bevorzugt ist R' Wasserstoff oder C₁-C₄-Alkyl, insbesondere Wasserstoff, Methyl oder Aethyl.

X steht z.B. für Halogen, wie Fluor, Chlor oder Brom. Sulfo, C₁-C₄-Alkylsulfonyl oder Phenylsulfonyl und bevorzugt für Halogen. insbesondere für Fluor oder Chlor.

Bedeutet T eine als Anion abspaltbare Gruppe, so handelt es sich hierbei z.B. um Halogen, wie Fluor, Chlor oder Brom, Sulfo, C₁-C₄-Alkylsulfonyl oder Phenylsulfonyl und bevorzugt um Halogen, insbesondere um Fluor oder Chlor.

Steht T für einen nicht-reaktiven Substituenten, so kann dieser z.B. Hydroxy, C₁-C₄-Alkoxy, C₁-C₄-Alkylthio, Morpholino oder gegebenenfalls substituiertes Amino sein.

Bevorzugt handelt es sich bei T um Halogen, Hydroxy, Sulfo, C₁-C₄-Alkoxy, C₁-C₄-Alkylthio, C₁-C₄-Alkylsulfonyl, Phenylsulfonyl, Morpholino oder gegebenenfalls substituiertes Amino.

Als gegebenenfalls substituiertes Amino kommt für T unsubstituiertes Amino oder beispielsweise N-C₁-C₄-Alkylamino oder N.N-Di-C₁-C₄-Alkylamino, wobei das Alkyl gegebenenfalls z.B. durch Sulfo, Sulfato, Hydroxy. Carboxy oder Phenyl substituiert ist, Cyclohexylamino, N-C₁-C₄-Alkyl-N-phenylamino oder Phenylamino oder Naphthylamino in Betracht, wobei das Phenyl oder Naphthyl gegebenenfalls z.B. durch C₁-C₄-Alkyl, C₁-C₄-Alkoxy, C₂-C₄-Alkanoylamino, Carboxy, Sulfo oder Halogen substituiert ist.

Beispiele für geeignete nicht-reaktive Substituenten T sind Amino, Methylamino, Äthylamino, β-Hydroxyäthylamino, N,N-Di-β-Hydroxyäthylamino, β-Sulfoäthylamino, Cyclohexylamino, Morpholino, o-, m- oder p-Chlorphenylamino, o-, m- oder p-Methylphenylamino, o-, m- oder p-Methoxyphenylamino, o-, m- oder p-Sulfophenylamino, Disulfophenylamino, o-Carboxyphenylamino, 1- oder 2-Naphthylamino, 1-Sulfo-2-naphthylamino, 4,8-Disulfo-2-naphthylamino, N-Äthyl-N-phenylamino, N-Methyl-N-phenylamino, Methoxy, Äthoxy, n- oder iso-Propoxy sowie Hydroxy.

Besonders bevorzugt hat T die Bedeutung Chlor, Fluor, Hydroxy, Sulfo, C₁-C₄-Alkoxy, C₁-C₄-Alkylthio, Morpholino, Amino, N-C₁-C₄-Alkylamino, das im Alkylteil unsubstituiert oder durch Hydroxy, Sulfato oder Sulfo substituiert ist, Phenylamino oder N-C₁-C₄-Alkyl-N-phenylamino, worin das Phenyl jeweils unsubstituiert oder durch Sulfo, Carboxy, Acetylamino, Methyl oder Methoxy substituiert ist.

Ganz besonders bevorzugt hat T die Bedeutung Chlor, Fluor, C₁-C₄-Alkoxy, Morpholino, N-C₁-C₄-Alkyl-N-phenylamino oder Phenylamino, worin das Phenyl jeweils unsubstituiert oder durch Sulfo, Carboxy, Acetylamino, Methyl oder Methoxy, insbesondere Sulfo, substituiert ist.

Bei dem als Anion abspaltbaren Rest X₁ handelt es sich vorzugsweise um Halogen, insbesondere um Fluor oder Chlor.

Handelt es sich bei X₁ um einen nicht-reaktiven Substituenten, so kommen z.B. die oben für T in der Bedeutung als nicht-reaktiver Substituent genannten Bedeutungen und Bevorzugungen in Betracht.

Besonders bevorzugt handelt es sich bei dem Rest X₁ um Halogen, insbesondere um Fluor oder Chlor.

Beispiele für geeignete Reste X₂ sind Nitro, Cyan, C₁-C₄-Alkylsulfonyl, Carboxy, Chlor, Hydroxy, C₁-C₄-Alkoxysulfonyl, C₁-C₄-Alkylsulfinyl, C₁-C₄-Alkoxycarbonyl oder C₂-C₄-Alkanoyl, wobei die Bedeutungen Chlor, Cyano und Methylsulfonyl für X₂ bevorzugt sind. Besonders bevorzugt ist X₂ Halogen, insbesondere Chlor.

Bevorzugt sind Reaktivreste der Formel (3), worin X Halogen und T Halogen, Hydroxy, Sulfo, C₁-C₄-Alkoxy, C₁-C₄-Alkylthio, C₁-C₄-Alkylsulfonyl, Phenylsulfonyl, Morpholino oder gegebenenfalls substituiertes Amino ist, wobei für T die oben angegebenen Bevorzugungen gelten.

Als Reaktivreste der Formel (4) sind solche bevorzugt, worin beide Substituenten X₁ Halogen, insbesondere Chlor oder Fluor, bedeuten und X₂ Halogen, insbesondere Chlor, ist.

Besonders bevorzugt als Reaktivreste der Formel (4) sind solche, worin beide Substituenten X₁ Fluor oder Chlor bedeuten und X₂ Chlor ist.

Als faserreaktive Gruppen enthalten die Reaktivfarbstoffe der Formel (1) bevorzugt einen Reaktivrest der Formeln (2b) bis (2g), oder einen Reaktivrest der Formel (3) oder (4), worin R' Wasserstoff oder unsubstituiertes oder durch Hydroxyl, Sulfo oder Sulfato substituiertes und mit Ausnahme von Methyl gegebenenfalls durch Sauerstoff unterbrochenes C₁-C₁₂-Alkyl ist oder der Rest -(NR')- einen bivalenten 5- bis 7-gliedrigen aliphatischen Heterocyclus darstellt, X, X₁ und X₂ Halogen bedeuten und T Halogen, Hydroxy, Sulfo, C₁-C₄-Alkoxy, C₁-C₄-Alkylthio, C₁-C₄-Alkylsulfonyl, Phenylsulfonyl, Morpholino oder gegebenenfalls substituiertes Amino ist, wobei für die Reaktivreste der Formeln (2b) bis (2g), (3) und (4) die oben angegebenen Bedeutungen und Bevorzugungen gelten.

Als C₁-C₄-Alkyl kommen für R₁ Methyl, Aethyl, Propyl, Isopropyl, Butyl, Isobutyl, sek.-Butyl oder tert.-Butyl, insbesondere Methyl, in Betracht.

Für R₂ ist die Bedeutung als Cyano oder Carbamoyl bevorzugt.

R₃ und R₄ bedeuten unabhängig voneinander Wasserstoff, gegebenenfalls durch Hydroxyl, Sulfo oder Sulfato substituiertes und mit Ausnahme von Methyl gegebenenfalls durch Sauerstoff unterbrochenes C₁-C₁₂-Alkyl,
oder einen Rest der Formel 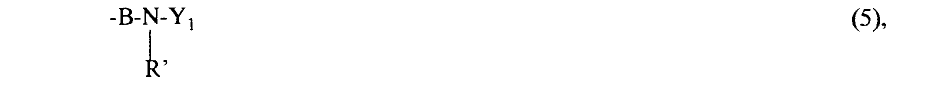 worin R' die unter Formel (1) angegebenen Bedeutungen hat, B gegebenenfalls durch Hydroxyl, Sulfo oder Sulfato substituiertes, und gegebenenfalls durch Sauerstoff unterbrochenes C₂-C₁₂-Alkylen und Y₁ ein faserreaktiver Rest der Halogentriazin- oder Halogenpyrimidinreihe ist, insbesondere ein Reaktivrest der Formel 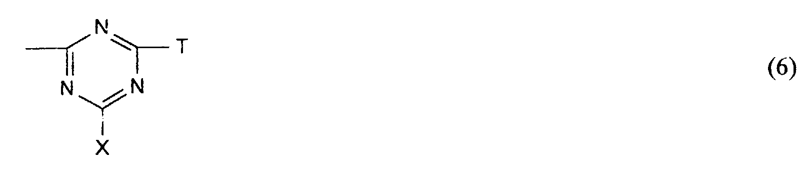 oder 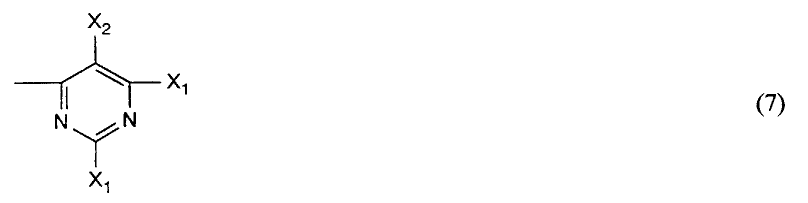 ist, wobei T, X, X₁ und X₂ die oben angegebenen Bedeutungen und Bevorzugungen haben.

Es kann sich somit bei den Resten R₃ und R₄ sowohl um nicht-reaktive Reste als auch um reaktive Reste handeln.

Die Reste B, R₃ und R₄ können durch Sauerstoff unterbrochen sein, wie z.B. durch 1 bis 3, insbesondere 1 oder 2 Reste -O-.

Der Rest B ist bevorzugt C₂-C₈-Alkylen, welches gegebenenfalls durch Hydroxyl, Sulfo oder Sulfato substituiert ist und gegebenenfalls durch 1 bis 3, insbesondere 1 oder 2 Reste -O- unterbrochen ist.

Besonders bevorzugt handelt es sich bei dem Rest B um C₂-C₆-Alkylen, insbesondere um 1,2-Aethylen, 1,3-Propylen oder 1,6-Hexylen.

Die Reste R₃ und R₄ sind als nicht-reaktive Reste bevorzugt Wasserstoff, gegebenenfalls durch Hydroxyl, Sulfo oder Sulfato substituiertes und mit Ausnahme von Methyl gegebenenfalls durch durch 1 bis 3, insbesondere 1 oder 2 Reste -O- unterbrochenes C₁-C₈-Alkyl.

Als Beispiele für nicht-reaktive Reste R₃ und R₄ seien Methyl, Aethyl, Propyl, Isopropyl, Butyl, Isobutyl, sek.-Butyl, tert.-Butyl oder geradkettiges oder verzweigtes Pentyl, Hexyl oder Octyl, 2-Hydroxyäthyl, 3-Hydroxypropyl, 2-Sulfatoäthyl, 3-Sulfatopropyl, 2-Sulfoäthyl, 3-Sulfopropyl. 2-Methoxyäthyl, 3-Methoxypropyl, sowie Reste der Formeln -(CH₂)₂-O-(CH₂)₂-OH und -(CH₂)₂-O-(CH₂)₂-OSO₃H genannt.

Als reaktive Reste R₃ und R₄ sind solche der Formel (5) bevorzugt, worin B und R' die unter Formel (5) angegebenen Bedeutungen haben, X Halogen und T Halogen, Hydroxy, Sulfo, C₁-C₄-Alkoxy, C₁-C₄-Alkylthio, C₁-C₄-Alkylsulfonyl, Phenylsulfonyl, Morpholino oder gegebenenfalls substituiertes Amino ist, beide Substituenten X₁ Halogen, insbesondere Chlor oder Fluor, bedeuten und X₂ Halogen, insbesondere Chlor, ist.

Ganz besonders bevorzugt als reaktive Reste R₃ und R₄ sind solche der Formel (5), worin R' Wasserstoff oder unsubstituiertes oder durch Hydroxyl, Sulfo oder Sulfato substituiertes und mit Ausnahme von Methyl gegebenenfalls durch 1 bis 3 Reste -O-unterbrochenes C₁-C₁₂-Alkyl, insbesondere C₁-C₈-Alkyl, ist, oder der Rest -(NR')- einen
Rest der Formel 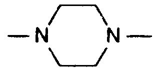 darstellt, B gegebenenfalls durch Hydroxyl, Sulfo oder Sulfato substituiertes und gegebenenfalls durch 1 bis 3 Reste -O- unterbrochenes C₂-C₈-Alkylen ist, X Fluor oder Chlor, T Fluor, Chlor, C₁-C₄-Alkoxy, Morpholino, N-C₁-C₄-Alkyl-N-phenylamino oder Phenylamino, worin das Phenyl jeweils unsubstituiert oder durch Sulfo, Carboxy, Acetylamino, Methyl oder Methoxy, insbesondere Sulfo, substituiert ist, beide Substituenten X₁ Fluor oder Chlor bedeuten und X₂ Chlor ist.

Der Rest D ist bevorzugt ein Rest der Benzol- oder Naphthalinreihe oder der Rest eines Monoazofarbstoffes, welcher eine Diazokomponente der Benzol- oder Naphthalinreihe und eine Kupplungskomponente der Benzol- oder Naphthalinreihe enthält, wobei die genannten Benzol- und Naphthalinreste gegebenenfalls durch C₁-C₄-Alkyl, C₁-C₄-Alkoxy, C₂-C₄-Alkanoylamino, Halogen. Sulfo oder einen Reaktivrest der Formeln (2b) bis (2g), (3) oder (4) substituiert sind.

Besonders bevorzugt sind Reaktivfarbstoffe der Formeln 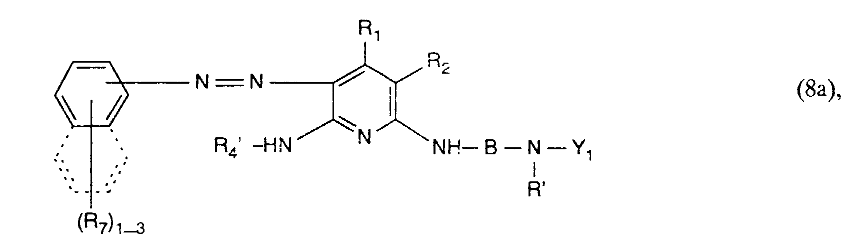 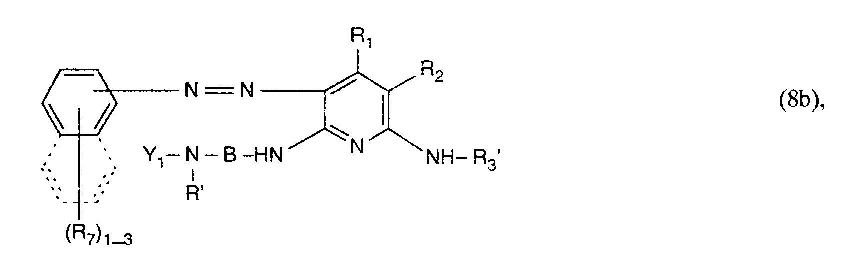 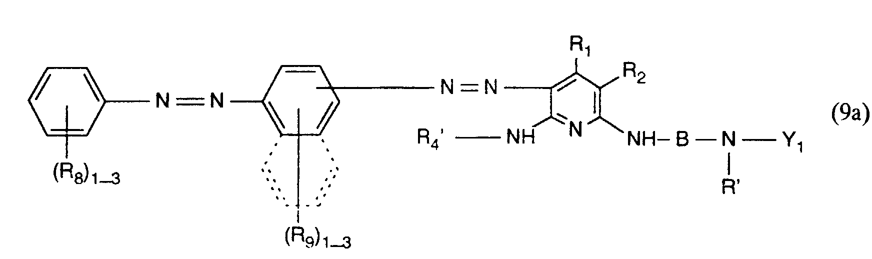 und 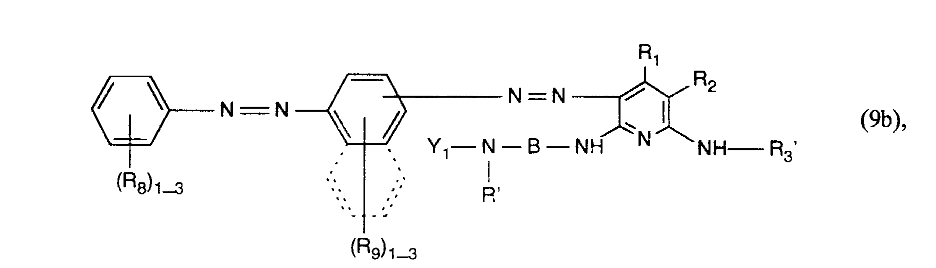 worin R₁ C₁-C₄-Alkyl, R₂ Cyano, Carbamoyl oder Sulfomethyl ist, R', R₃' und R₄' unabhängig voneinander Wasserstoff oder gegebenenfalls durch Hydroxyl, Sulfo oder Sulfato substituiertes und mit Ausnahme von Methyl gegebenenfalls durch Sauerstoff unterbrochenes C₁-C₁₂-Alkyl bedeuten, der Rest -(NR')- einen bivalenten 5- bis 7-gliedrigen aliphatischen Heterocyclus darstellen kann, (R₇)₁₋₃, (R₈)₁₋₃ und (R₉)₁₋₃ jeweils für 1 bis 3 gleiche oder verschiedene Substituenten aus der Gruppe Wasserstoff, C₁-C₄-Alkyl, C₁-C₄-Alkoxy, C₂-C₄-Alkanoylamino, Halogen und Sulfo stehen, B gegebenenfalls durch Hydroxyl, Sulfo oder Sulfato substituiertes und gegebenenfalls durch Sauerstoff unterbrochenes C₂-C₁₂-Alkylen und Y₁ ein faserreaktiver Rest der Formel (6) oder (7) ist.

Ebenfalls besonders bevorzugt sind Reaktivfarbstoffe der Formeln 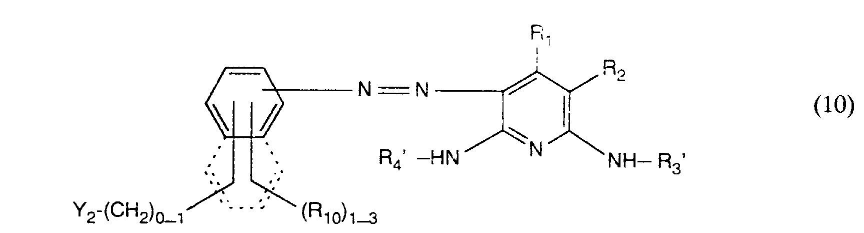 und 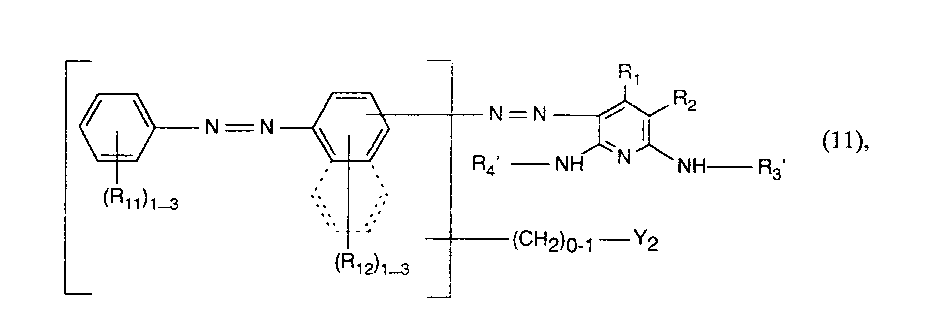 worin R₁ C₁-C₄-Alkyl, R₂ Cyano, Carbamoyl oder Sulfomethyl ist, R₃' und R₄' unabhängig voneinander Wasserstoff oder gegebenenfalls durch Hydroxyl, Sulfo oder Sulfato substituiertes und mit Ausnahme von Methyl gegebenenfalls durch Sauerstoff unterbrochenes C₁-C₁₂-Alkyl bedeuten, (R₁₀)₁₋₃, (R₁₁)₁₋₃ und (R₁₂)₁₋₃ jeweils für 1 bis 3 gleiche oder verschiedene Substituenten aus der Gruppe Wasserstoff, C₁-C₄-Alkyl, C₁-C₄-Alkoxy, C₂-C₄-Alkanoylamino, Halogen und Sulfo stehen und Y₂ ein faserreaktiver Rest der Formeln (2b) bis (2g), (3) oder (4) ist.

Für die Reste R₁, R₂, R', B, Y₁ als Rest der Formel (6) oder (7) und Y₂ als Rest der Formeln (2b) bis (2g), (3) oder (4) gelten hierbei die oben angegebenen Bedeutungen und Bevorzugungen. Für die Reste R₃' und R₄' gelten die für R₃ und R₄ in der Bedeutung als nichtreaktive Reste angegebenen Bevorzugungen.

Besonders bevorzugte Reaktivreste sind solche der Formeln (2b) bis (2g), (3) und (4), insbesondere solche der Formeln (2b), (2e), (2g), (3) und (4) und vorzugsweise solche der Formeln (3) und (4).

Gegenstand der Erfindung sind ferner Mischungen von Reaktivfarbstoffen, welche dadurch gekennzeichnet sind, dass sie mindestens zwei Reaktivfarbstoffe der Formel (1) enthalten, wobei für die Reaktivfarbstoffe der Formel (1) die oben angegebenen Bedeutungen und Bevorzugungen gelten.

Reaktivfarbstoffe der Formel (1), worin R₃ und R₄ voneinander verschiedene Bedeutungen haben, liegen in der Regel als Mischungen von zwei Reaktivfarbstoffen vor, welche sich lediglich dadurch unterscheiden, dass die Positionen der Reste R₃ und R₄ vertauscht sind. Besonders bevorzugt sind somit Mischungen, welche einen Reaktivfarbstoff der Formel 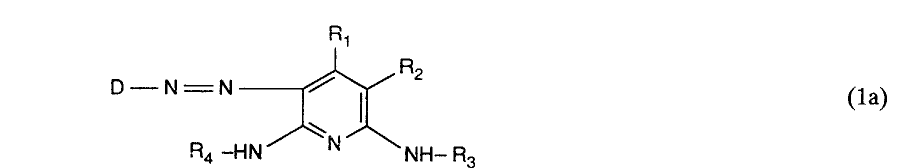 und einen Reaktivfarbstoff der Formel 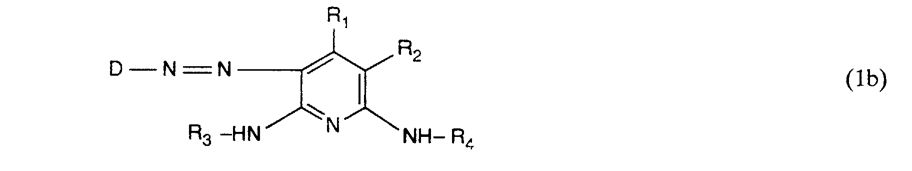 enthalten, wobei sich die Reaktivfarbstoffe der Formeln (1a) und (1b) nur dadurch unterscheiden, dass die Positionen der Reste R₃ und R₄ vertauscht sind.

Ein weiterer Gegenstand der vorliegenden Erfindung ist ein Verfahren zur Herstellung der Reaktivfarbstoffe der Formel (1), welches dadurch gekennzeichnet ist, dass man ein Amin der Formel

D-NH₂ (12),

worin D die unter Formel (1) angegebenen Bedeutungen hat, diazotiert und auf eine Kupplungskomponente der Formel 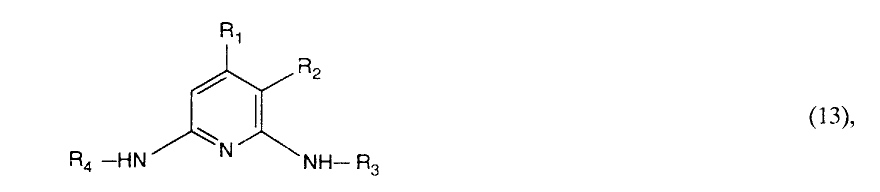 worin R₁, R₂, R₃ und R₄ die unter Formel (1) angegebenen Bedeutungen haben, kuppelt, und gegebenenfalls eine weitere Umwandlungsreaktion anschliesst.

Die Diazotierung des Amins der Formel (12) erfolgt in der Regel durch Einwirken salpetriger Säure in wässrig-mineralsaurer Lösung bei tiefer Temperatur, wie z.B. 0 bis 10°C, die Kupplung auf die Kupplungskomponente der Formel (13) bei sauren, neutralen bis schwach alkalischen pH-Werten, insbesondere bei einem pH-Wert von 2 bis 8.

Eine abgewandelte Ausführungsform des Verfahrens besteht darin, dass man zunächst einen Farbstoff herstellt, der eine Vorstufe des Reaktivrestes enthält, und diese nachträglich in die Endstufe umwandelt, z.B. durch Veresterung oder eine Additionsreaktion. Beispielsweise kann man einen Farbstoff, worin Z ein Rest HO-CH₂CH₂- ist, herstellen und dieses Produkt mit Schwefelsäure umsetzen, so dass die Hydroxygruppe in die Sulfatogruppe überführt wird. Die Sulfatierung der Hydroxygruppe erfolgt z.B. durch Umsetzung mit konzentrierter Schwefelsäure bei ca. 0°C bis mässig erhöhter Temperatur.

Ausserdem können Eliminierungsreaktionen an die Synthese angeschlossen werden. Beispielsweise kann man Reaktivfarbstoffe der Formel (1), welche Sulfatoäthylsulfonylreste enthalten, mit einer Base, wie z.B. Natriumhydroxid, behandeln, wobei die Sulfatoäthylsulfonylreste in Vinylsulfonylreste übergehen.

Grundsätzlich lassen sich die Reaktivfarbstoffe der Formel (1) herstellen, indem man von Vorprodukten oder Zwischenprodukten für Farbstoffe, die faserreaktive Reste enthalten, ausgeht, oder diese faserreaktiven Reste in hierfür geeignete Zwischenprodukte mit Farbstoffcharakter einführt.

Ein weiteres interessantes Verfahren zur Herstellung von Reaktivfarbstoffen der Formel (1) ist dadurch gekennzeichnet, dass man eine Verbindung der Formel 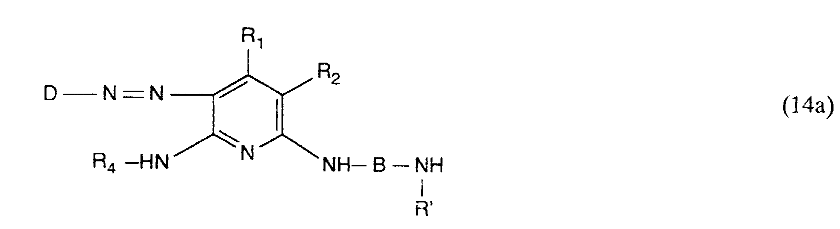 oder 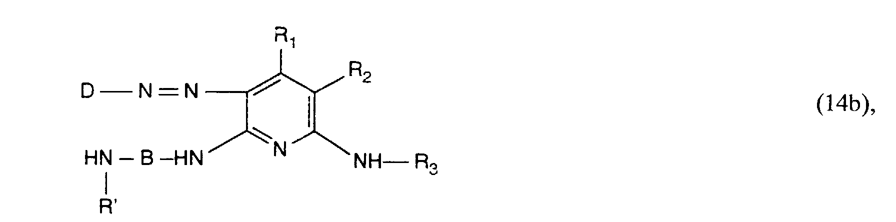 worin R', B, D, R₁, R₂, R₃ und R₄ die unter Formel (1) angegebenen Bedeutungen haben, wobei die Verbindungen der Formeln (14a) und (14b) keinen faserreaktiven Rest enthalten, mit einem faserreaktiven Rest der Halogentriazin- oder Halogenpyrimidinreihe kondensiert.

Die Kondensation erfolgt in der Regel in wässriger Lösung, bei einer Temperatur von beispielsweise 0 bis 50°C und einem pH von z.B. 4 bis 9.

Für die Substituenten der Verbindungen der Formeln (12), (13), (14a) und (14b) gelten die oben angegebenen Bevorzugungen.

Als faserreaktive Reste der Halogentriazin- oder Halogenpyrimidinreihe, welche mit den Verbindungen der Formeln (14a) und (14b) kondensiert werden, kommen insbesondere Reste der Formeln 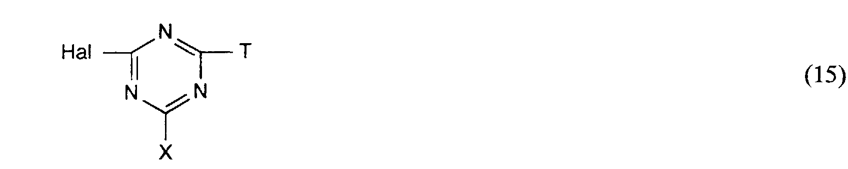 oder 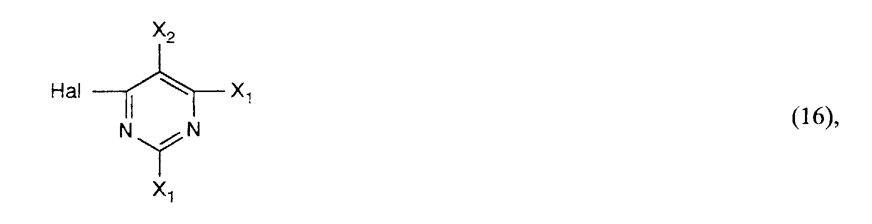 worin Hal Halogen, insbesondere Chlor oder Fluor, bedeutet und T, X, X₁ und X₂ die oben angegebenen Bedeutungen und Bevorzugungen haben, in Betracht.

Die Verbindungen der Formeln (14a) und (14b) können durch Diazotierung eines entsprechenden Amins der Formel (12) und Kupplung auf eine Kupplungskomponente der Formel 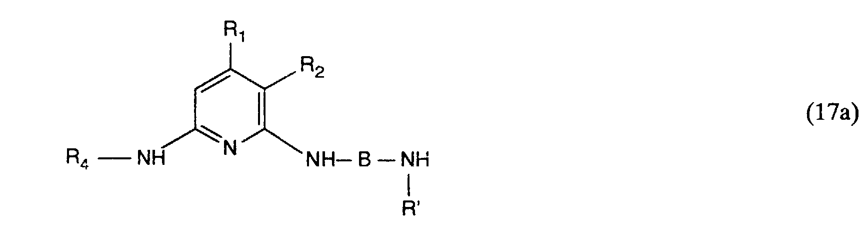 oder 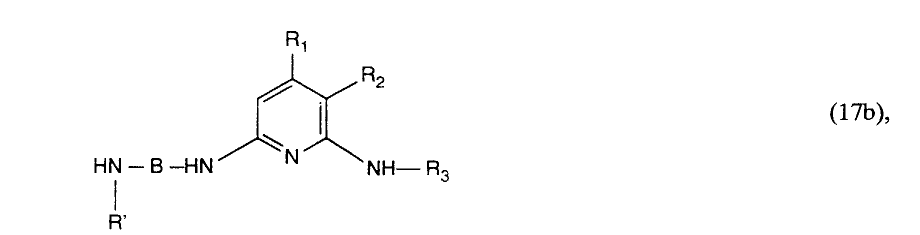 analog wie für die Diazotierung des Amins der Formel (12) und Kupplung auf die Kupplungskomponente der Formel (13) angegeben, erhalten werden.

Die Verbindungen der Formeln (12), (13), (15) und (16) sind bekannt oder können in Analogie zu bekannten Verbindungen hergestellt werden.

Ein Verfahren zur Herstellung der Verbindungen der Formeln (17a) und (17b) ist dadurch gekennzeichnet, dass man eine Verbindung der Formel 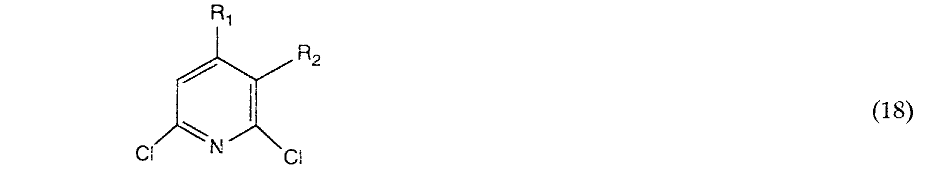 mit Aminen der Formeln

NH₂-R₁₃ (19)

und

NH₂-B-NH-R' (20)

umsetzt und gegebenenfalls eine weitere Umwandlungsreaktion anschliesst, wobei B, R', R₁ und R₂ die oben angegebenen Bedeutungen haben und R₁₃ die für R₃ und R₄ angegebenen Bedeutungen hat.

Die Umsetzung erfolgt beispielsweise bei einer Temperatur von ca. 70 bis 120°C, insbesondere 70 bis 90°C, in einem Lösungsmittel, wie z.B. N,N-Diäthylanilin, Triäthylamin, höheren Alkoholen oder Ketonen, oder man verwendet die Amine der Formeln (19) und (20) selbst als Lösungsmittel.

Verwendet man Mischungen der Amine der Formeln (19) und (20) bei vollständigem Austausch der Chloratome, so werden in der Regel Mischungen der Verbindungen der Formeln (17a) und (17b) erhalten.

Durch geeignete Wahl der Reaktionsbedingungen können die Chloratome der Verbindung der Formel (18) jedoch stufenweise substituiert werden, was die Isolierung der einzelnen Mono- oder Disubstitutionsprodukte erlaubt.

So wird in einem ersten Schritt bei niedrigerer Temperatur, wie z.B. 10 bis 50°C, vorwiegend das sich in para-Stellung zum Rest R₂ befindende Chlor-Atom abgespalten, während in einem zweiten Schritt bei höherer Temperatur, wie z.B. 70 bis 120°C, das sich in ortho-Stellung zum Rest R₂ befindende Chlor-Atom abgespalten wird.

Werden somit die Amine der Formeln (19) und (20) nicht zusammen, sondern nacheinander zugegeben, so kann entweder die Verbindung der Formel (17a) oder die Verbindung der Formel (17b) als Hauptprodukt erhalten werden. Hierbei hat es sich als vorteilhaft erwiesen, das nach Reaktion des im ersten Schritt bei niedrigerer Temperatur erhaltene monosubstituierte Zwischenprodukt zu isolieren und danach den zweiten Schritt, die Umsetzung des zweiten Amins bei höherer Temperatur, auszuführen.

Wird daher im ersten Schritt bei niedriger Temperatur das Amin der Formel (19) und im zweiten Schritt bei höherer Temperatur das Amin der Formel (20) zugegeben, so wird als Hauptverbindung die Verbindung der Formel (17a) erhalten. Werden die Amine in umgekehrter Reihenfolge eingesetzt, so wird als Hauptverbindung die Verbindung der Formel (17b) erhalten.

Als vorteilhaft hat es sich erwiesen, das Amin der Formel (20) in grossem Ueberschuss einzusetzen.

Die erfindungsgemässen Reaktivfarbstoffe der Formel (1) sowie die Verbindungen der Formeln (17a) und (17b), welche eine Sulfo- oder Sulfatogruppe enthalten, liegen entweder in der Form ihrer freien Säure oder vorzugsweise als deren Salze vor.

Als Salze kommen beispielsweise die Alkali-, Erdalkali- oder Ammoniumsalze oder die Salze eines organischen Amins in Betracht. Als Beispiele seien die Natrium-, Lithium-, Kalium- oder Ammoniumsalze oder das Salz des Mono-, Di- oder Triäthanolamins genannt.

Die Reaktivfarbstoffe der Formel (1) eignen sich zum Färben und Bedrucken der verschiedensten Materialien, wie hydroxylgruppenhaltigen oder stickstoffhaltigen Fasermaterialien. Als Beispiele seien Seide, Leder, Wolle, Polyamidfasern und Polyurethane, und insbesondere cellulosehaltige Fasermaterialien aller Art genannt. Solche cellulosehaltige Fasermaterialien sind beispielsweise die natürlichen Cellulosefasern, wie Baumwolle, Leinen und Hanf, sowie Zellstoff und regenerierte Cellulose. Die Reaktivfarbstoffe der Formel (1) sind auch zum Färben oder Bedrucken von hydroxylgruppenhaltigen Fasern geeignet, die in Mischgeweben enthalten sind, z.B. von Gemischen aus Baumwolle mit Polyesterfasern oder Polyamidfasern. Besonders geeignet sind die Reaktivfarbstoffe der Formel (1) zum Färben oder Bedrucken von cellulosehaltigen Fasermaterialien oder insbesondere natürlichen oder synthetischen Polyamidfasermaterialien.

Die erfindungsgemässen Farbstoffe lassen sich auf verschiedene Weise auf das Fasermaterial applizieren und auf der Faser fixieren, insbesondere in Form von wässrigen Farbstofflösungen und -druckpasten. Sie eignen sich sowohl für das Ausziehverfahren als auch zum Färben nach dem Foulard-Färbeverfahren, wonach die Ware mit wässrigen, gegebenenfalls salzhaltigen Farbstofflösungen imprägniert wird, und die Farbstoffe nach einer Alkalibehandlung oder in Gegenwart von Alkali, gegebenenfalls unter Wärmeeinwirkung, fixiert werden. Besonders geeignet sind sie für das sogenannte Kaltverweilverfahren, wonach der Farbstoff zusammen mit dem Alkali auf dem Foulard aufgebracht wird und danach durch mehrstündiges Lagern bei Raumtemperatur fixiert wird. Nach dem Fixieren werden die Färbungen oder Drucke mit kaltem und heissem Wasser, gegebenenfalls unter Zusatz eines dispergierend wirkenden und die Diffusion der nicht fixierten Anteile fördernden Mittels gründlich gespült.

Die Reaktivfarbstoffe der Formel (1) zeichnen sich durch hohe Reaktivität, gutes Fixiervermögen und ein sehr gutes Aufbauvermögen aus. Sie können daher nach dem Ausziehfärbeverfahren bei niedrigen Färbetemperaturen eingesetzt werden und erfordern beim Pad-Steam-Verfahren nur kurze Dämpfzeiten. Die Fixiergrade sind hoch und die nicht fixierten Anteile können leicht ausgewaschen werden, wobei die Differenz zwischen Ausziehgrad und Fixiergrad bemerkenswert klein, d.h. der Seifverlust sehr gering ist. Die Reaktivfarbstoffe der Formel (1) eignen sich auch besonders zum Druck, vor allem auf Baumwolle, ebenso aber auch zum Bedrucken von stickstoffhaltigen Fasern, z.B. von Wolle oder Seide oder von Mischgeweben, die Wolle oder Seide enthalten.

Die mit den erfindungsgemässen Farbstoffen hergestellten Färbungen und Drucke besitzen eine hohe Farbstärke und eine hohe Faser-Farbstoff-Bindungsstabilität, sowohl in saurem als auch in alkalischem Bereich, weiterhin eine gute Lichtechtheit und sehr gute Nassechtheitseigenschaften, wie Wasch-, Wasser-, Seewasser-, Ueberfärbe- und Schweissechtheiten, sowie eine gute Plissierechtheit, Bügelechtheit und Reibechtheit.

Die nachfolgenden Beispiele dienen zur Erläuterung der Erfindung. Die Temperaturen sind in Celsiusgraden angegeben, Teile sind Gewichtsteile, die Prozentangaben beziehen sich auf Gewichtsprozente, sofern nicht anders vermerkt. Gewichtsteile stehen zu Volumenteilen im Verhältnis von Kilogramm zu Liter.

Beispiel 1: 187 Teile 2,6-Dichlor-3-cyan-4-methylpyridin werden bei einer Temperatur von 20 bis 30°C in 427 Teile Aethanolamin eingetragen. Man rührt das Gemisch 2 Stunden bei 20 bis 30°C. Die klare braune Lösung wird auf 3000 Teile Eiswasser ausgetragen. Der entstandene Niederschlag wird abfiltriert, mit Wasser gewaschen und getrocknet.

Man erhält 180 Teile eines Gemisches der Verbindungen der Formeln 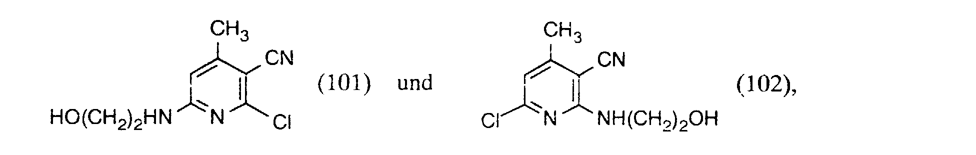 wobei das Verhältnis der Verbindung der Formel (101) zur Verbindung der Formel (102) 3:1 ist.

180 Teile des wie angegeben erhaltenen Gemisches der Verbindungen der Formeln (101) und (102) werden bei einer Temperatur von 100°C in 630 Teile 1,3-Diaminopropan eingetragen. Man rührt eine Stunde bei einer Temperatur von 100°C nach. Zur klaren Lösung streut man bei dieser Temperatur 45 Teile Natriumcarbonat zu. Unter reduziertem Vakuum wird das überschüssige 1,3-Diaminopropan abdestilliert. Der Rückstand wird mit 500 Teilen Wasser versetzt und die organische Phase bei Raumtemperatur abgetrennt. Zur Kristallisation wird die organische Phase in 600 Teilen Isopropanol gelöst und mit 85 Teilen Salzsäure (37%) angesäuert. Man rührt bei einer Temperatur von 0 bis 5°C und filtriert den entstandenen Niederschlag ab. Nach dem Trocknen erhält man 125 Teile eines Gemisches der Verbindungen der Formeln 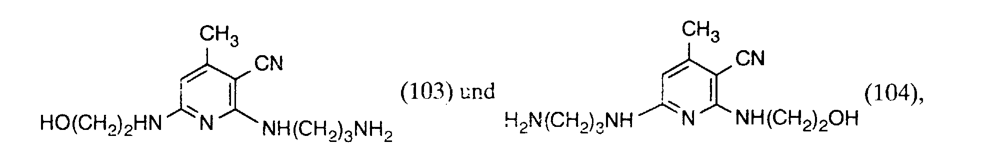 wobei das Verhältnis der Verbindung der Formel (103) zur Verbindung der Formel (104) 3:1 ist.

Beispiel 2: 187 Teile 2.6-Dichlor-3-cyan-4-methyl-pyridin werden bei einer Temperatur von 20 bis 30°C in 630 Teile 1,3-Diaminopropan eingetragen. Man rührt das Gemisch 2 Std. bei 20 bis 30°C.
Die braune Suspension wird abfiltriert und mit Eiswasser gewaschen und getrocknet. Man erhält ca. 150 Teile eines Gemisches bestehend aus

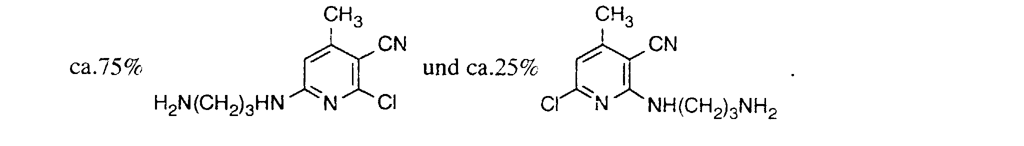

150 Teile des obigen Gemisches werden bei 100°C in 427 Teile Ethanolamin eingetragen. Man rührt 1 Std. bei 100 - 110°C nach. Zur klaren braunen Lösung streut man 45 Teile Natriumcarbonat zu. Unter Vakuum wird das überschüssige Ethanolamin abdestilliert, der Rückstand mit 500 Teilen Wasser versetzt und die organische Phase bei Raumtemperatur abgetrennt. Zur Kristallisation wird die organische Phase in 600 Teilen Isopropanol gelöst und mit 85 Teilen Salzsäure 37% angesäuert. Man rührt bei 0 bis 5°C und filtriert den entstandenen Niederschlag ab. Nach dem Trocknen erhält man ca. 110 Teile eines Gemisches aus

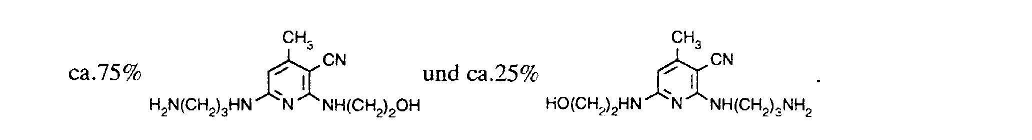

### Beispiele 3 bis 50:

Analog zu Beispiel 1 können Mischungen von 2,6-Diaminopyridinverbindungen erhalten werden, die die in der folgenden Tabelle 1 in Spalte 2 angegebene 2,6-Diaminopyridinverbindung als Haupkomponente enthalten. Die Komponenten der Mischungen unterscheiden sich nur dadurch, dass die Bedeutungen der Aminoreste in 2- und 6-Stellung vertauscht sind.

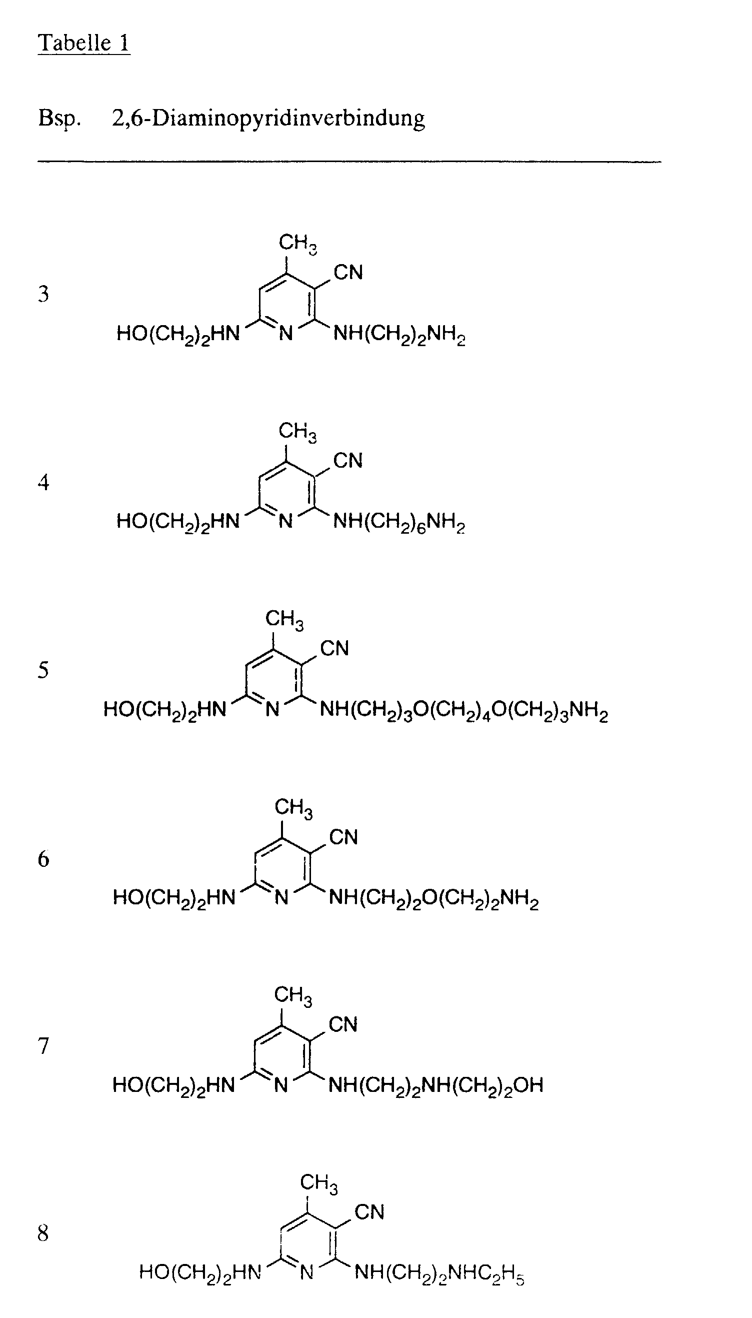

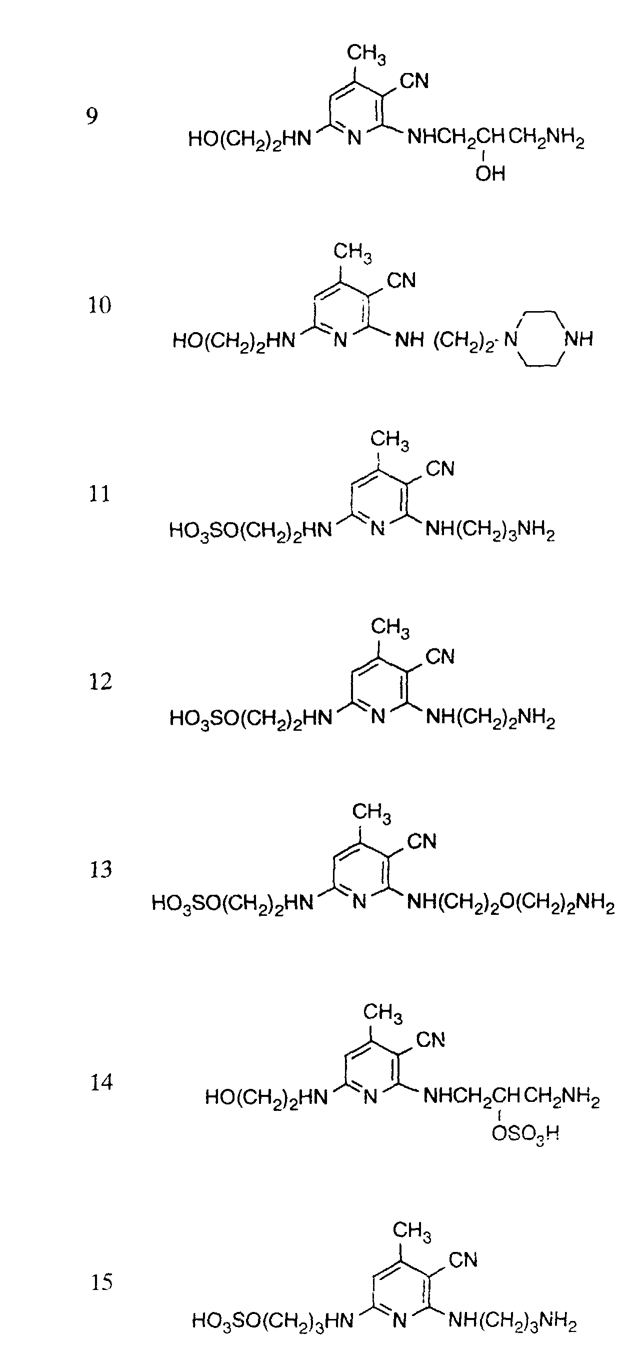

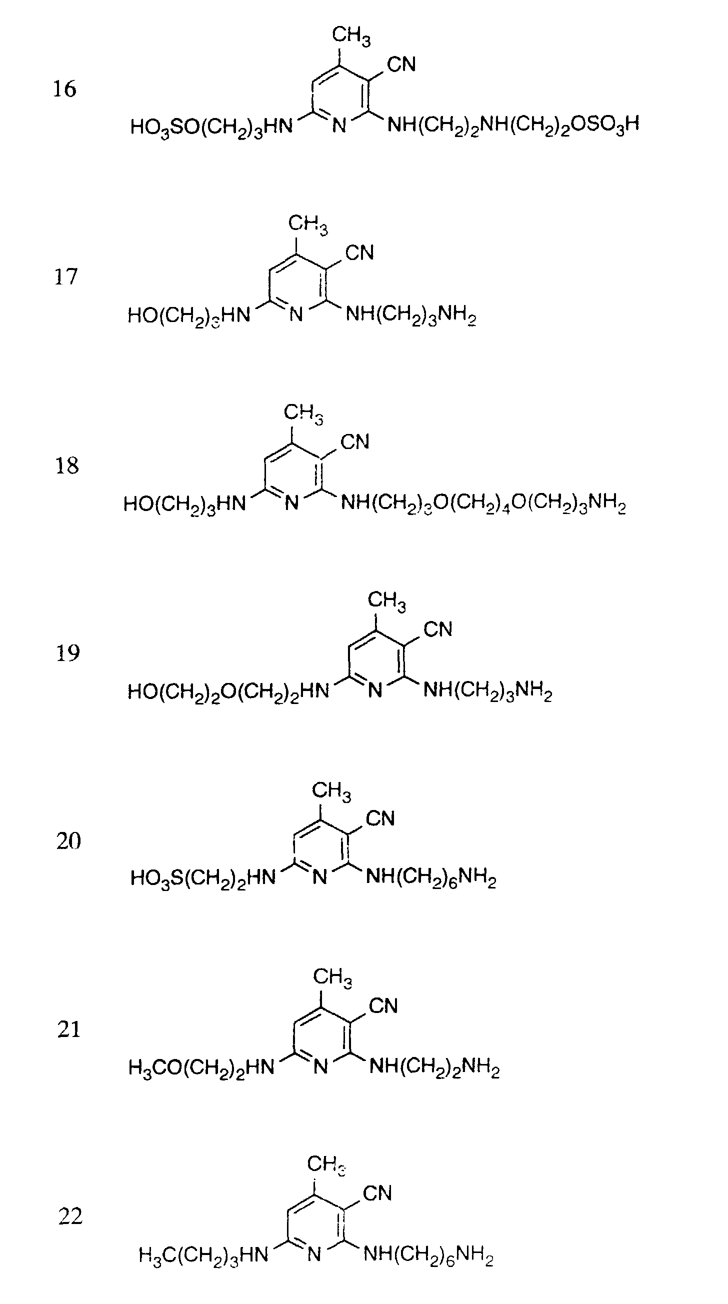

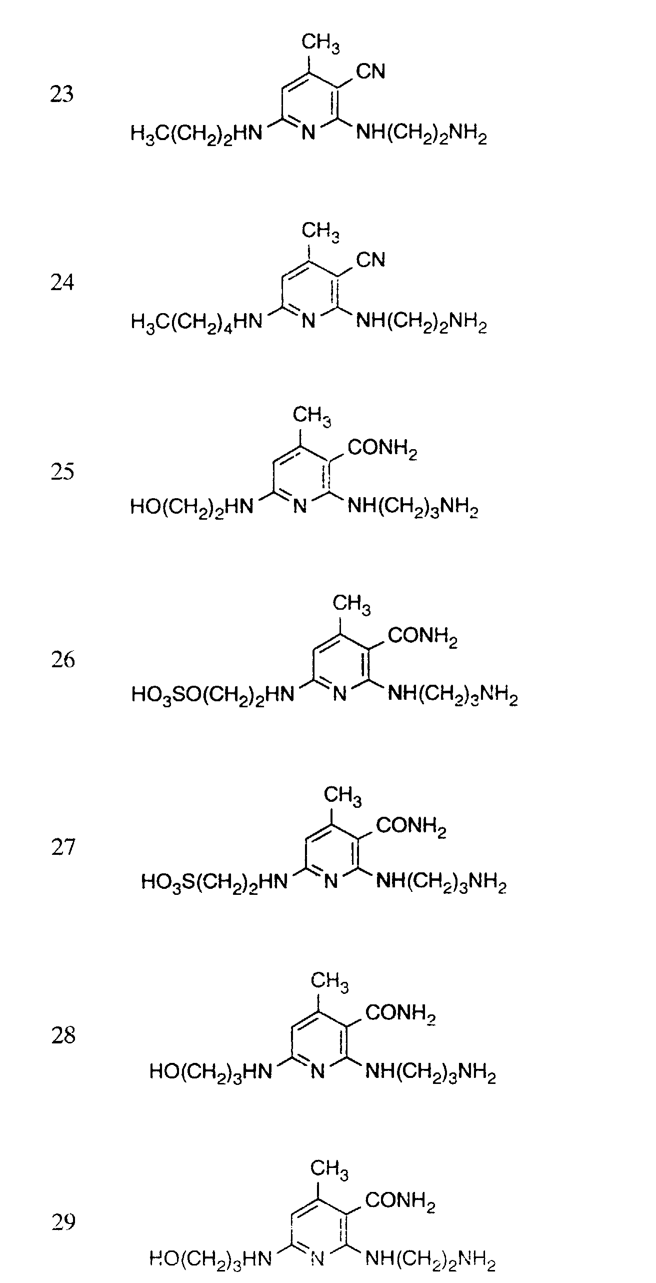

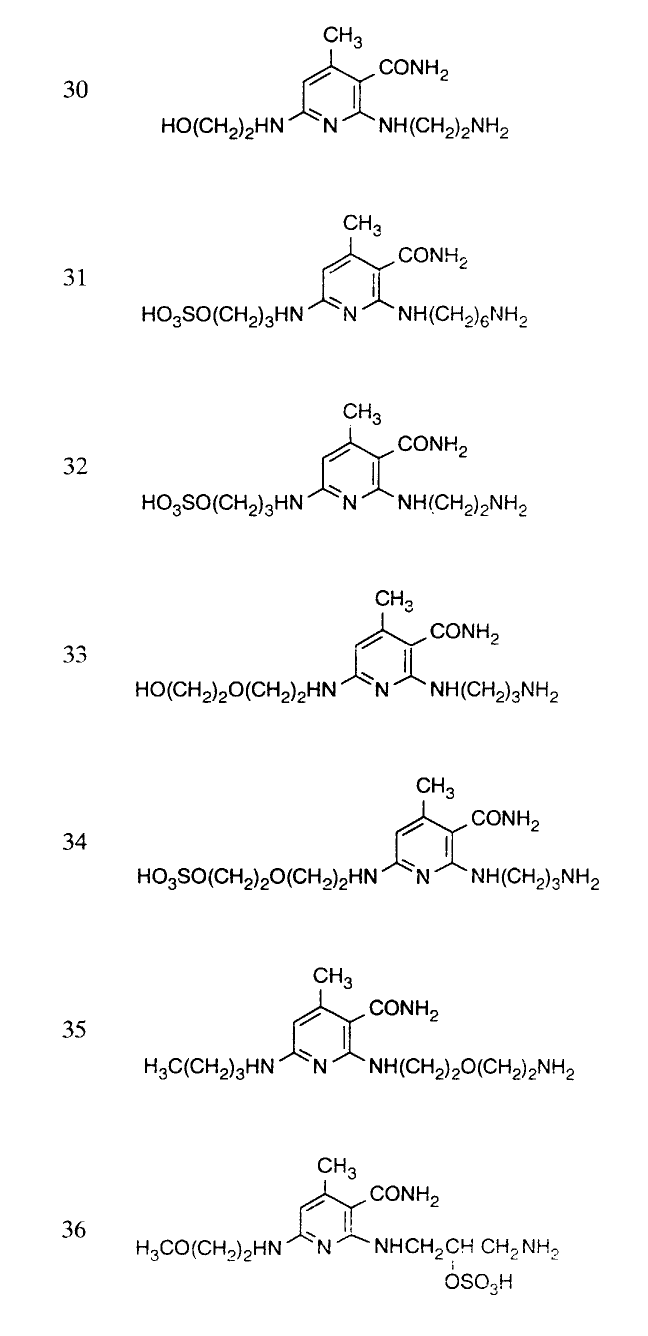

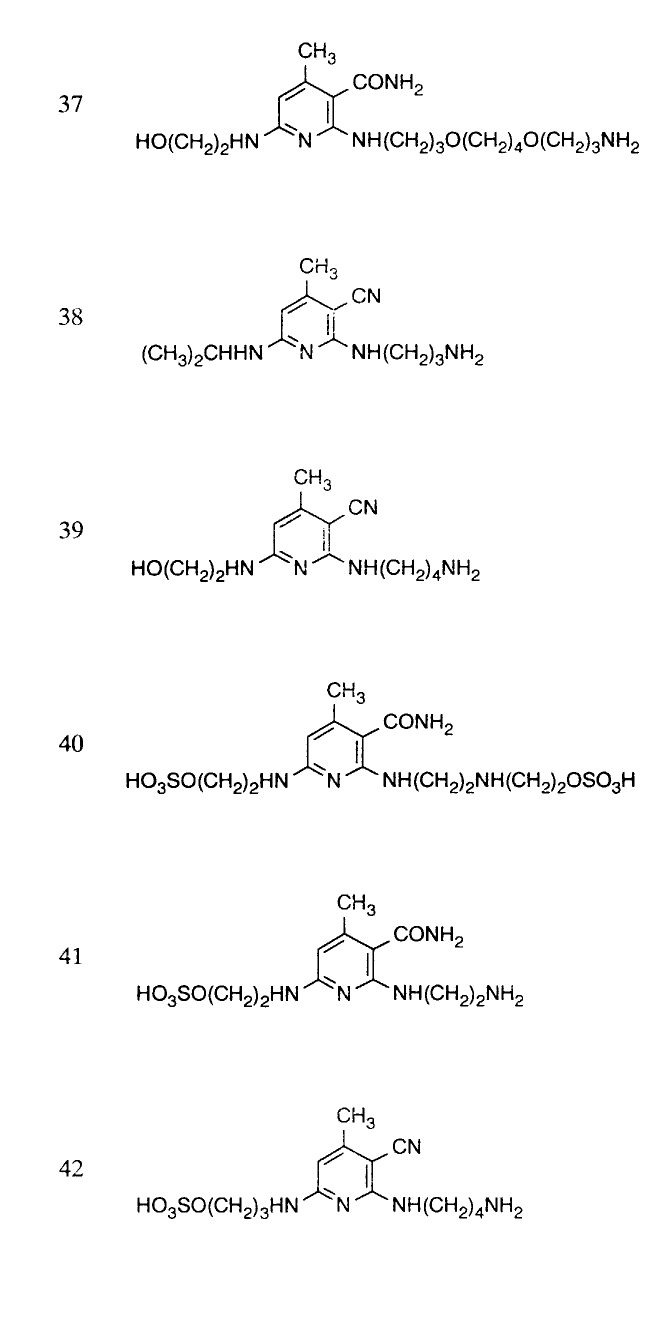

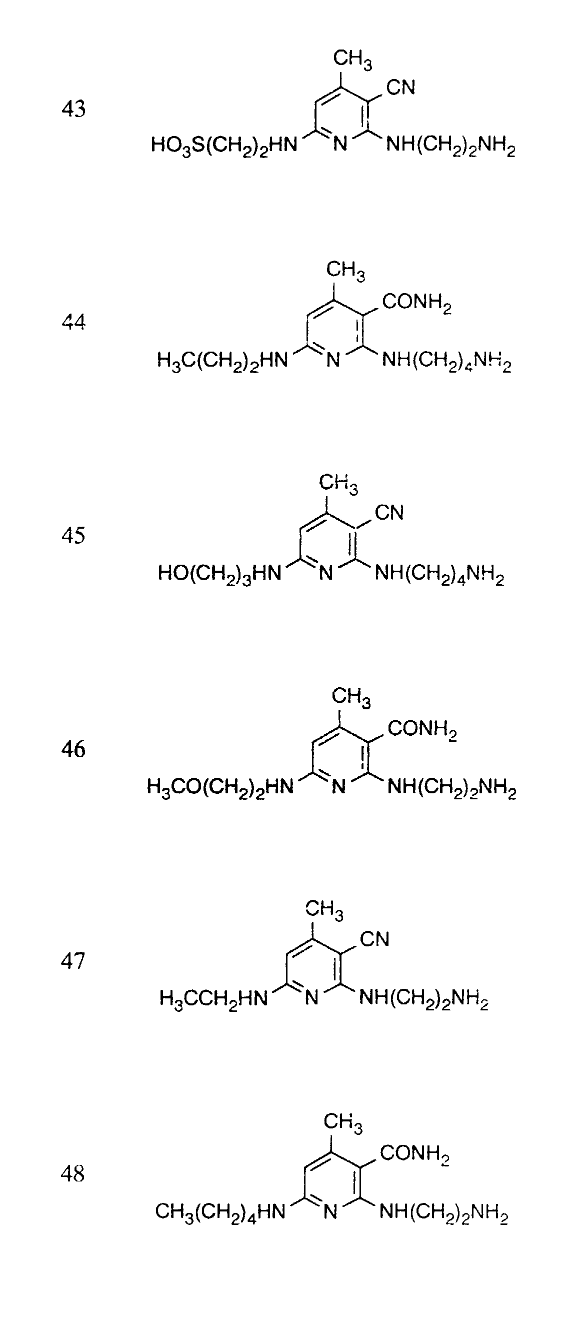

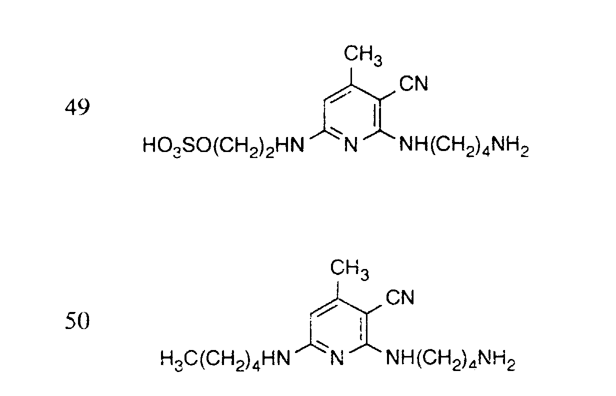

Beispiel 51: 22 Teile 5-(2',3'-Dibrompropionamido)anilin-2-sulfonsäure in 280 Teilen Eiswassersuspension werden mit 18 Teilen konzentrierter wässriger Salzsäure angesäuert und mit 15,6 Teilen einer 5-normalen Natriumnitritlösung diazotiert. Man rührt eine Stunde bei einer Temperatur von ca. 5 bis 15°C nach und zerstört dann überschüssige salpetrige Säure mittels Amidosulfonsäure. Die so hergestellte Diazoniumsalzlösung lässt man bei einem pH-Wert von 4 bis 5 langsam in eine Suspension aus 12 Teilen der Kupplungskomponente der Formel (105) in 100 Teilen Wasser einlaufen. Der pH wird durch Zugabe von Natriumbicarbonat auf einen Wert von 6 bis 7 erhöht. Man rührt 2 Stunden bis zur vollständigen Kupplung nach. Dann wird der Farbstoff umkehrosmotisiert und gefriergetrocknet. Man erhält einen Farbstoff, der in Form der freien Säure der Verbindung der Formel 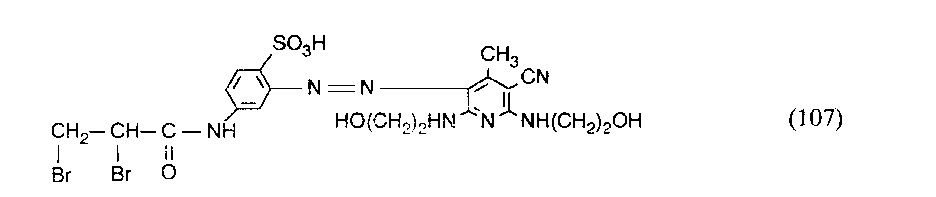 entspricht. Der Farbstoff der Formel (107) färbt Baumwolle und Wolle in brillanten gelben Tönen.

Beispiel 52: 22 Teile 4-(2',3'-Dibrompropionamido)anilin-2-sulfonsäure in 300 Teilen Eiswassersuspension werden mit 18 Teilen konzentrierter wässriger Salzsäure angesäuert und mit 15,6 Teilen einer 5-normalen Natriumnitritlösung diazotiert. Man rührt eine Stunde bei einer Temperatur von ca. 5 bis 15°C nach und zerstört dann überschüssige salpetrige Säure mittels Amidosulfonsäure. Die so hergestellte Diazoniumsalzlösung lässt man bei einem pH-Wert von 4 bis 5 langsam in eine Suspension aus 12 Teilen der Kupplungskomponente der Formel (105) in 100 Teilen Wasser einlaufen. Der pH wird durch Zugabe von Natriumbicarbonat auf einen Wert von 6 bis 7 erhöht. Man rührt 2 Stunden bis zur vollständigen Kupplung nach. Dann wird der Farbstoff umkehrosmotisiert und gefriergetrocknet. Man erhält einen Farhstoff, der in Form der freien Säure der Verbindung der Formel 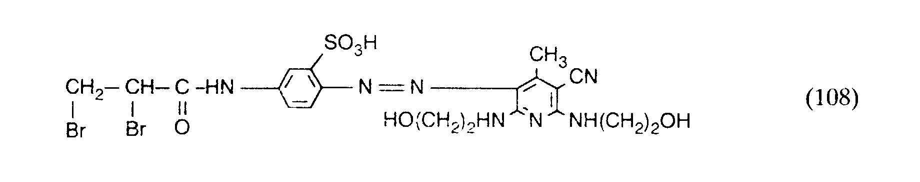 entspricht. Der Farbstoff der Formel (108) färbt Baumwolle und Wolle in brillanten orangen Tönen.

Beispiel 53: 25,3 Teile Anilin-2,5-disulfonsäure werden in 90 Teilen Wasser, durch Zusatz von Sodalösung bis zum pH-Wert von 4, gelöst. Zu der Lösung lässt man bei einer Temperatur von 0 bis 5°C 14 Teile Cyanurfluorid in 10 Minuten gleichmässig zutropfen, hält durch Zusatz von weiterer Sodalösung den pH-Wert auf 4 bis 4,5, rührt anschliessend 15 Minuten nach und stellt dann den pH-Wert auf 5,5.

Zu dieser Lösung lässt man dann eine Lösung aus 300 Teilen Wasser und 46 Teilen einer Mischung der Verbindungen der Formeln 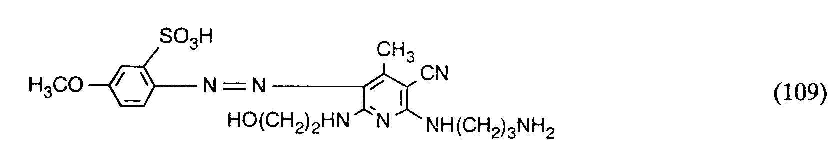 und 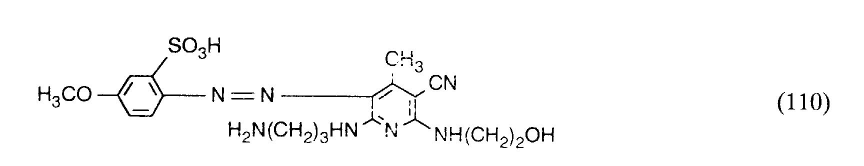 zulaufen und kondensiert bei Raumtemperatur und einem pH-Wert von 5,5 bis 8. Nach der Kondensation wird die Lösung umkehrosmotisiert und gefriergetrocknet. Man erhält 80 Teile einer Mischung, welche die in Form der freien Säuren angegebenen Farbstoffe der Formeln 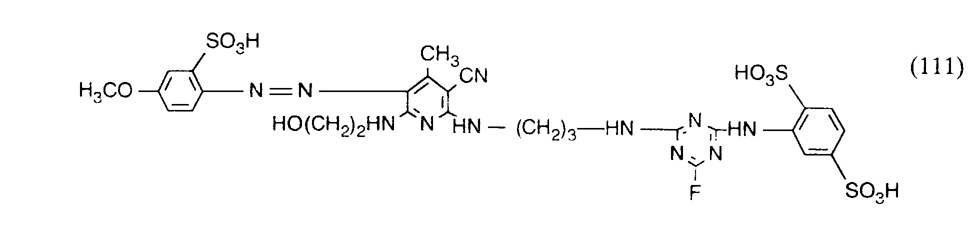 und 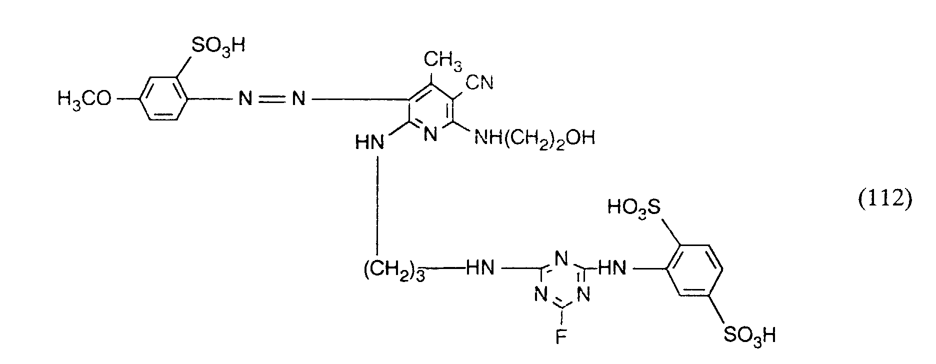 enthält, wobei der Farbstoff der Formel (111) die Hauptkomponente ist.

Die Mischung der Farbstoffe der Formeln (111) und (112) färbt Baumwolle und Wolle in brillanten orangen Tönen.

Die Mischung der Verbindungen der Formeln (109) und (110) kann durch Diazotierung von 2-Sulfo-4-methoxyanilin (durch Ansäuern mit Salzsäure und Zugabe von Natriumnitrit) und anschliessende Kupplung auf eine Mischung der gemäss Beispiel 1 erhältlichen Verbindungen der Formeln (103) und (104) hergestellt werden.

Beispiel 54: 25,3 Teile Anilin-2,5-disulfonsäure in 300 Teilen Eiswassersuspension werden, durch Zusatz einer Natriumbicarbonatlösung bis zum pH-Wert von 4, gelöst. Zu der Lösung lässt man eine Lösung von 19 Teilen Cyanurchlorid in Aceton innerhalb von 30 Minuten einlaufen und hält den pH-Wert durch Zusatz weiterer Natriumbicarbonatlösung bei 4 bis 4,5. Nach vollständiger Kondensation, bis kein freies Amin mehr durch Diazotierprobe nachweisbar ist, wird eine Lösung aus 18,8 Teilen 1,3-Phenylendiamin-4-sulfonsäure und 200 Teilen Wasser bei einem pH-Wert von 4 bis 5 zugegeben. Bei Raumtemperatur und einem pH-Wert von 4 bis 5 wird anschliessend 3 Stunden nachgerührt. Die so erhaltene Lösung enthält die in Form der freien Säure der Formel 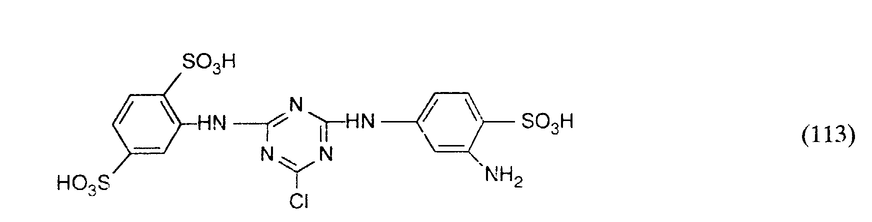 entsprechende Verbindung. Die die Verbindung der Formel (113) enthaltende Lösung wird in üblicher Weise diazotiert (durch Ansäuern mit Salzsäure und Zugabe von Natriumnitrit) und bei einer Temperatur von 0 bis 5°C und einem pH-Wert von 5 bis 8 auf eine Suspension aus 200 Teilen Wasser und 24 Teilen einer Kupplungskomponente der Formel 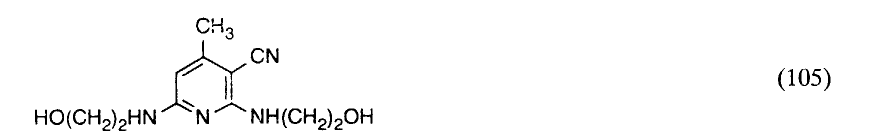 gekuppelt. Nach der Kupplung wird die Reaktionsmasse umkehrosmotisiert und gefriergetrocknet. Man erhält einen Farbstoff, der in Form der freien Säure der Verbindung der Formel 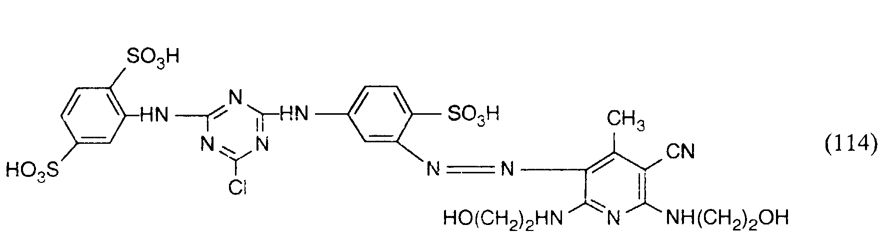 entspricht. Der Farbstoff der Formel (114) färbt Baumwolle und Wolle in brillanten goldgelben Tönen.

Beispiele 55 bis 124: Analog zu den Beispielen 51 bis 54 können die in Tabelle 2 in Form der freien Säuren angegebenen Reaktivfarbstoffe erhalten werden. Enthalten die in der Tabelle 2 angegebenen Reaktivfarbstoffe einen 2,6-Diaminopyridinrest, worin die in 2-und 6-Stellung gebundenen Aminoreste voneinander verschiedene Bedeutungen haben, so werden Mischungen von Reaktivfarbstoffen erhalten. welche die in der folgenden Tabelle 2 in Spalte 2 angegebenen Reaktivfarbstoffe als Haupkomponente enthalten. Die Komponenten der Mischungen unterscheiden sich nur dadurch, dass die Bedeutungen der Aminoreste in 2- und 6-Stellung des 2,6-Diaminopyridinrestes vertauscht sind. Die in Spalte 2 angegebenen Reaktivfarbstoffe und Reaktivfarbstoffmischungen färben Baumwolle und Wolle in den in Spalte 3 angegebenen Farbtönen.

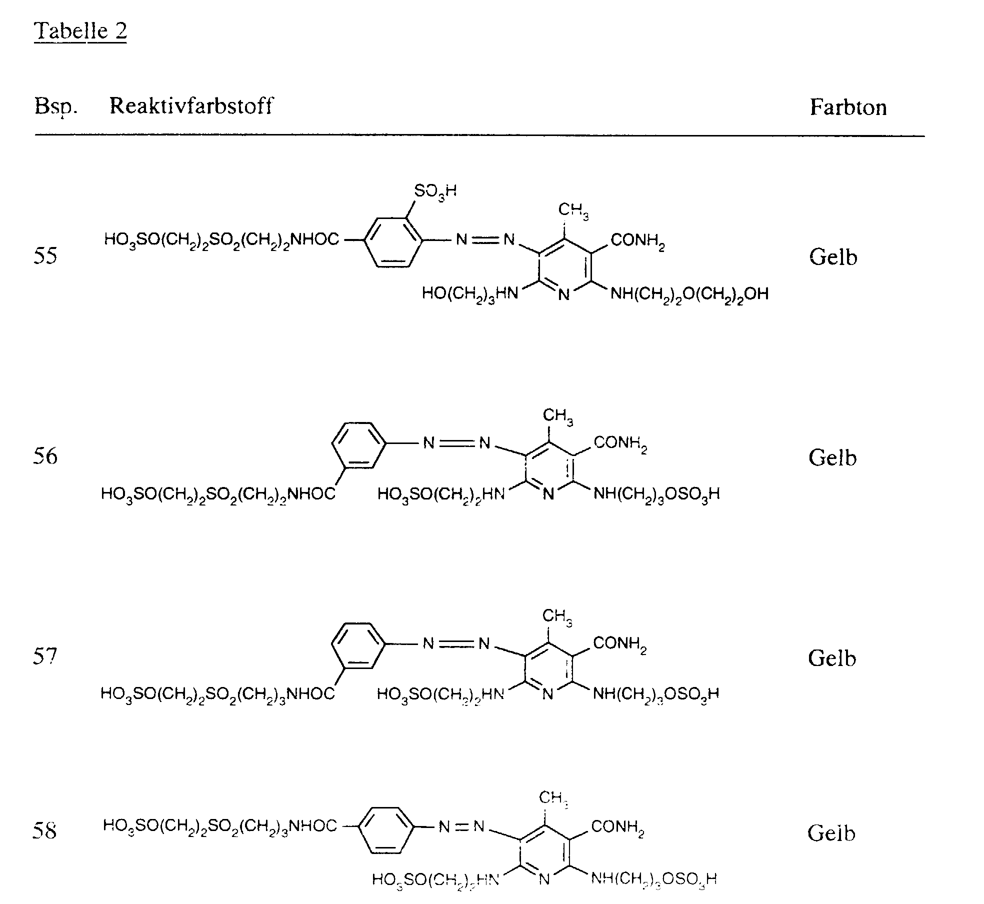

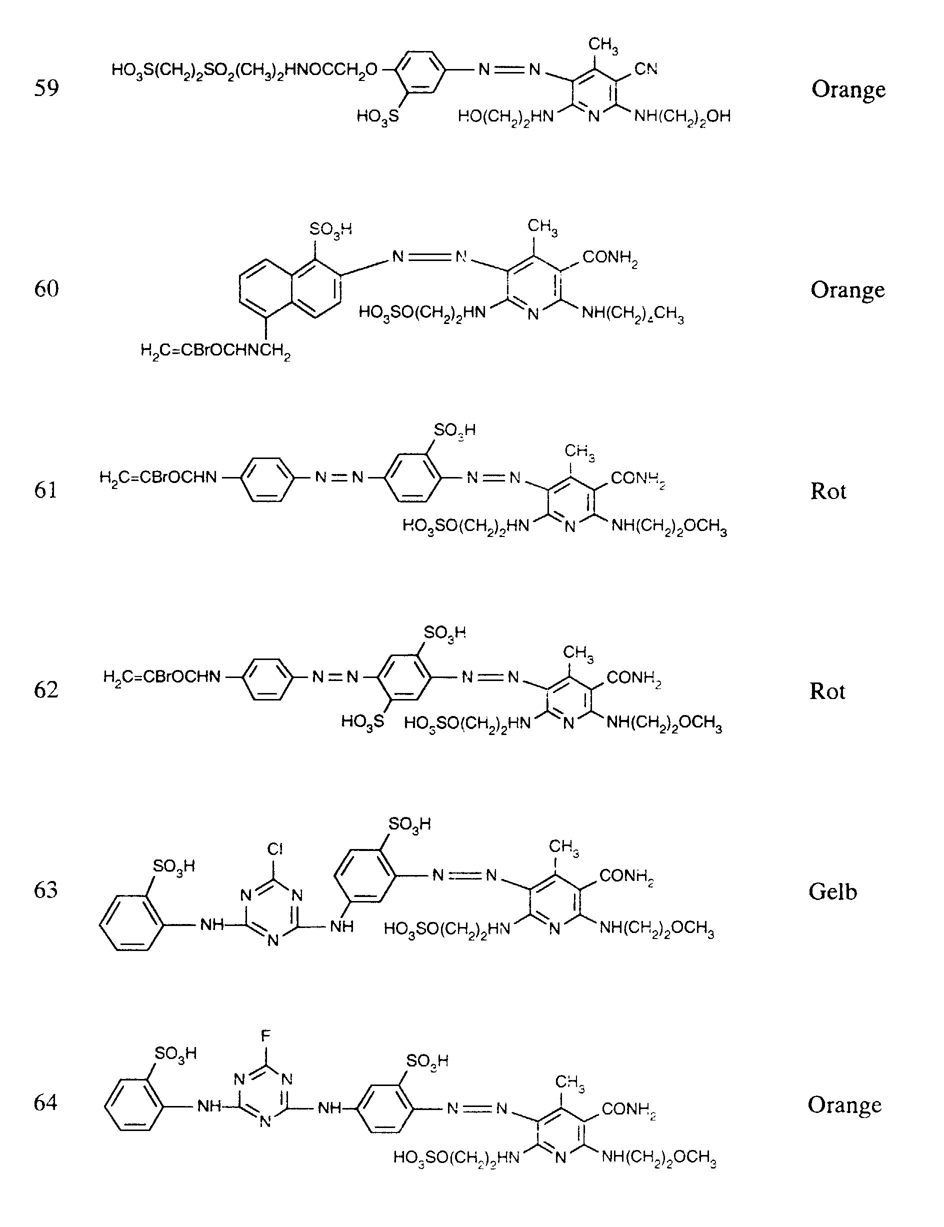

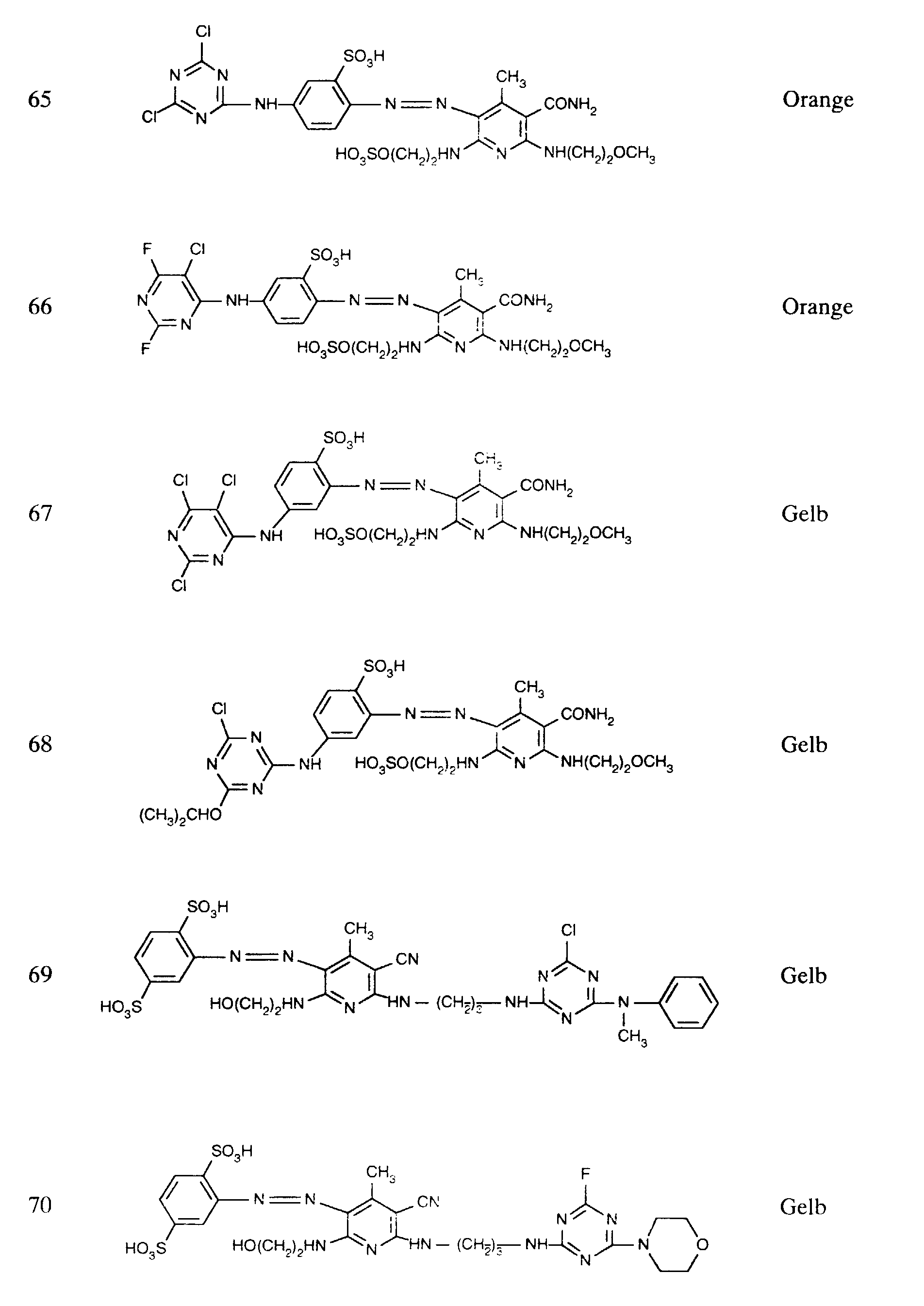

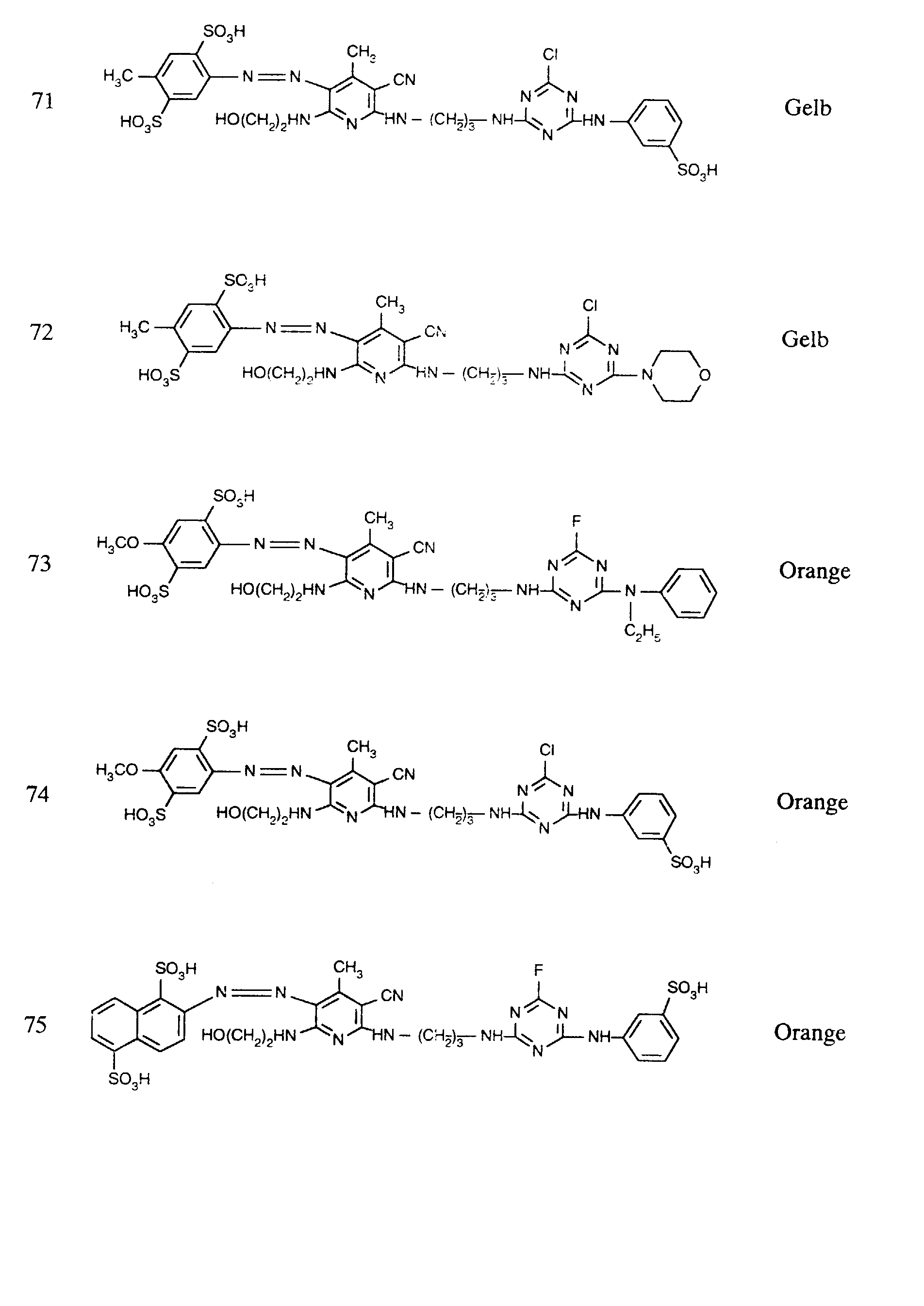

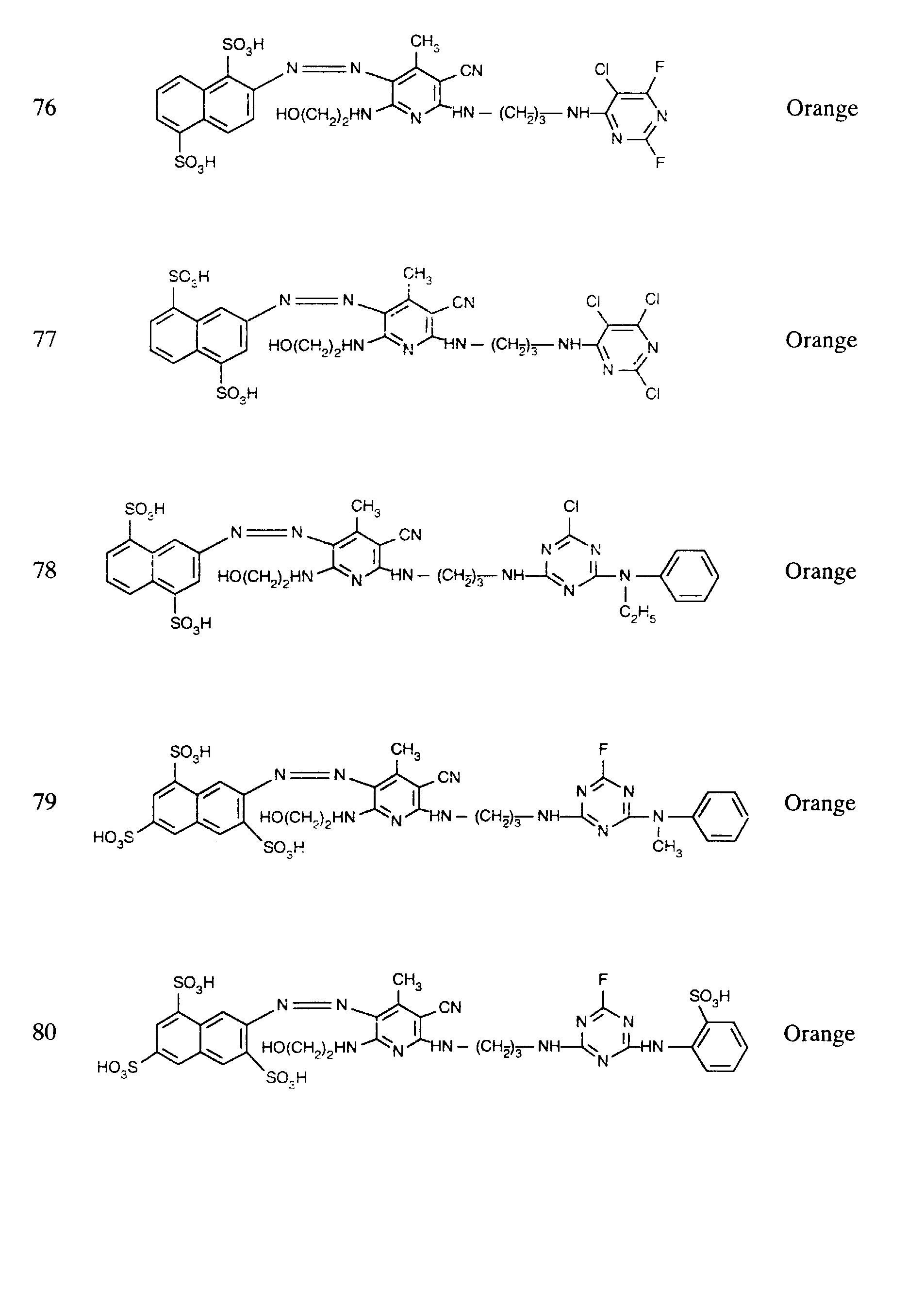

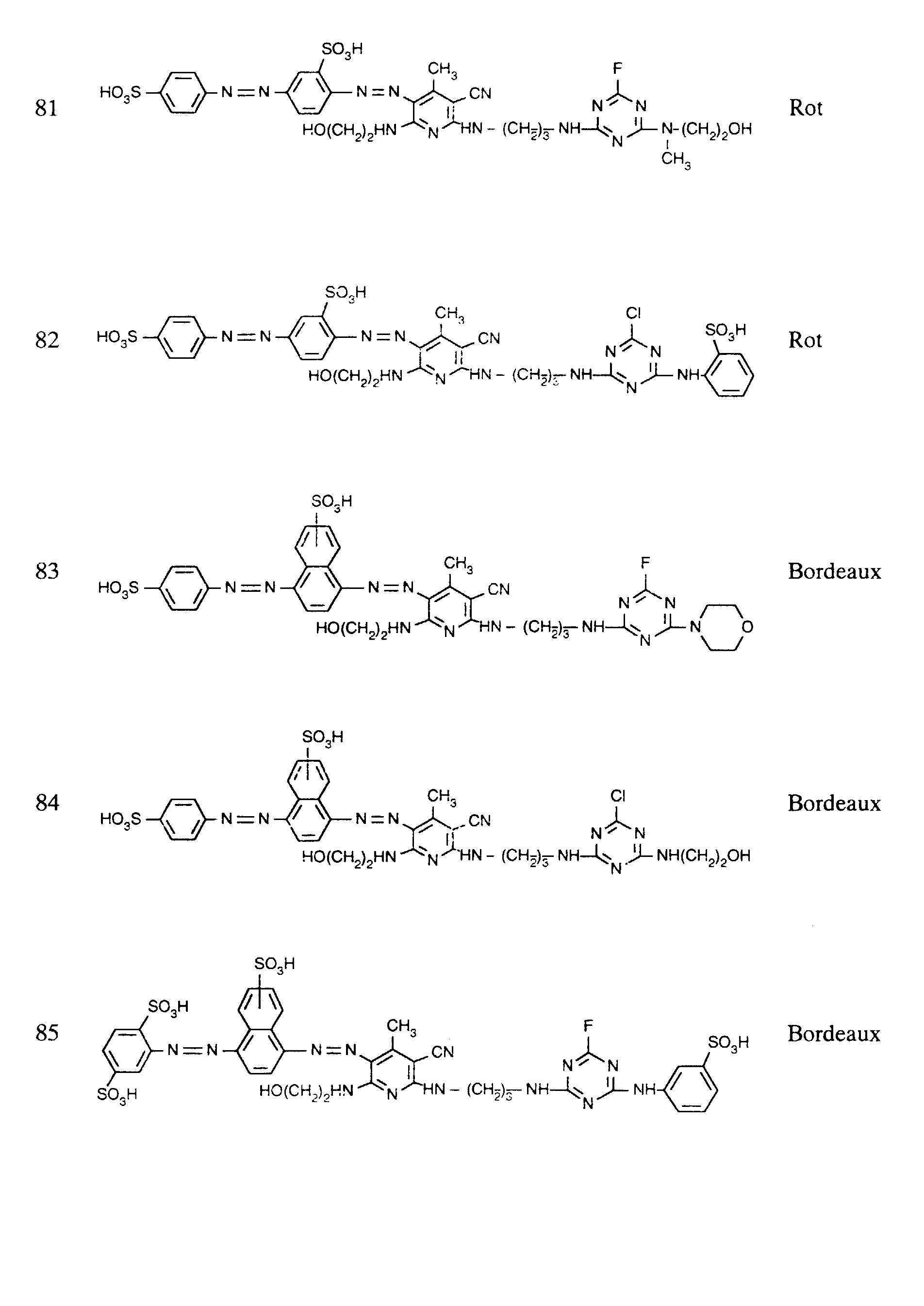

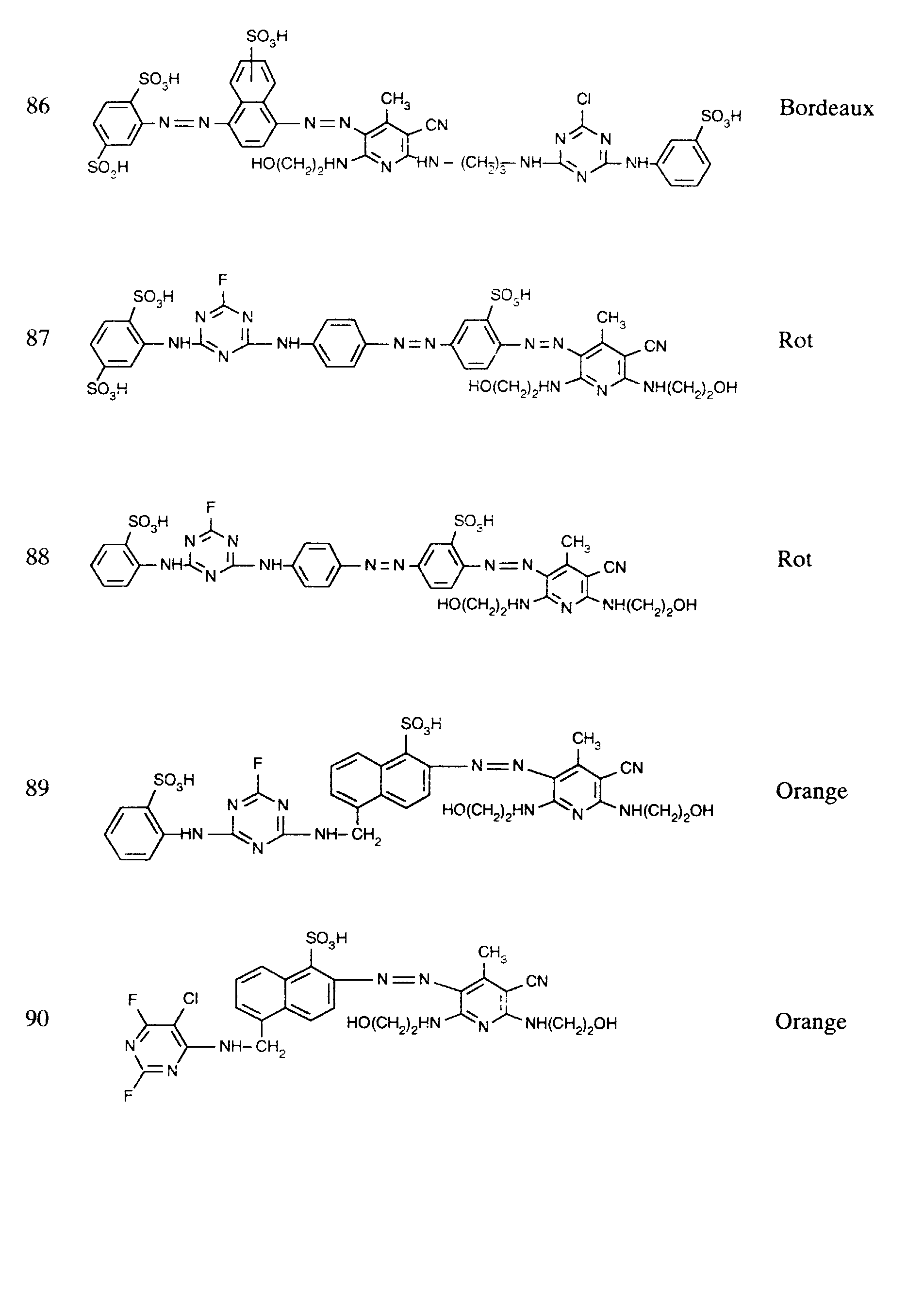

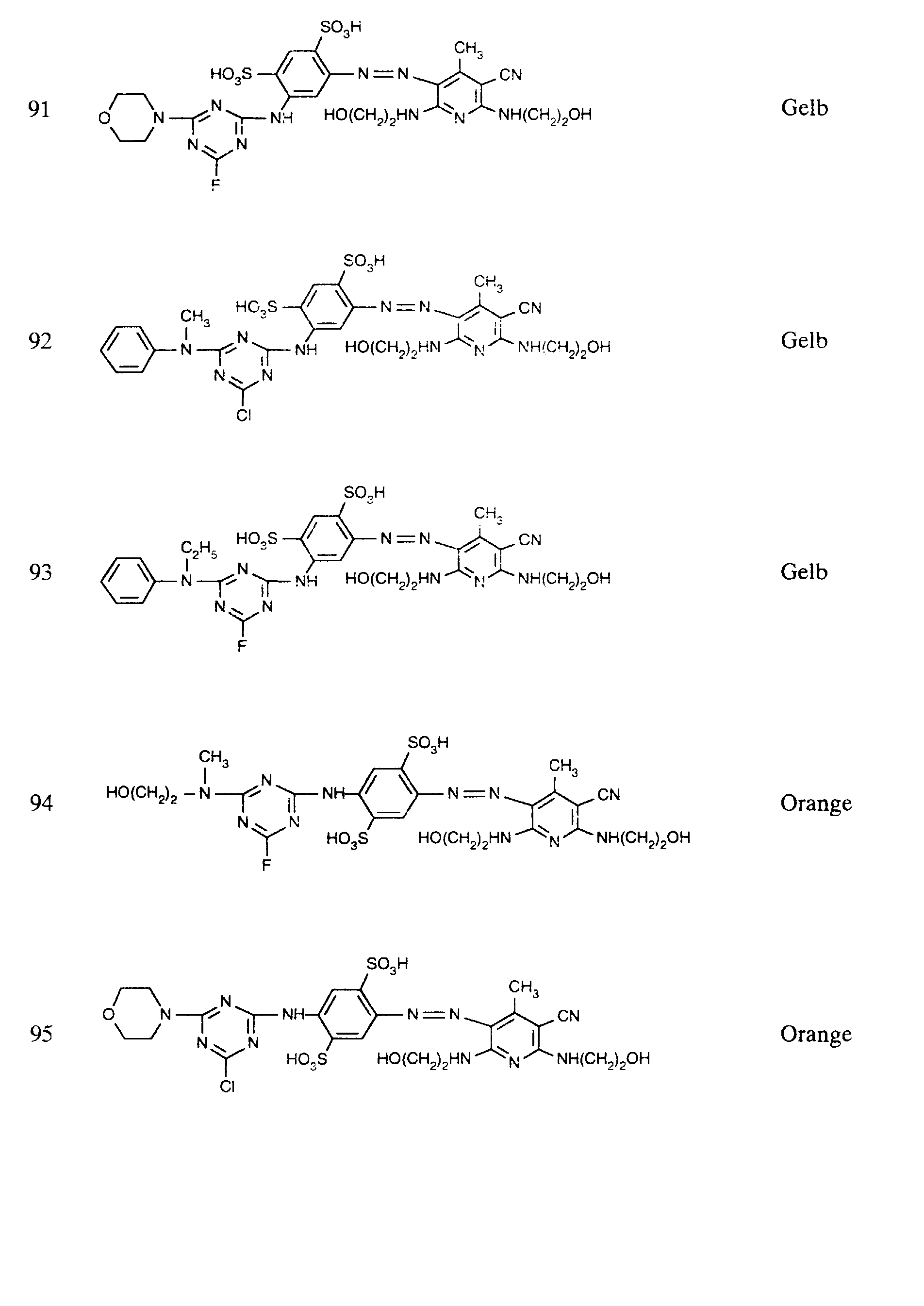

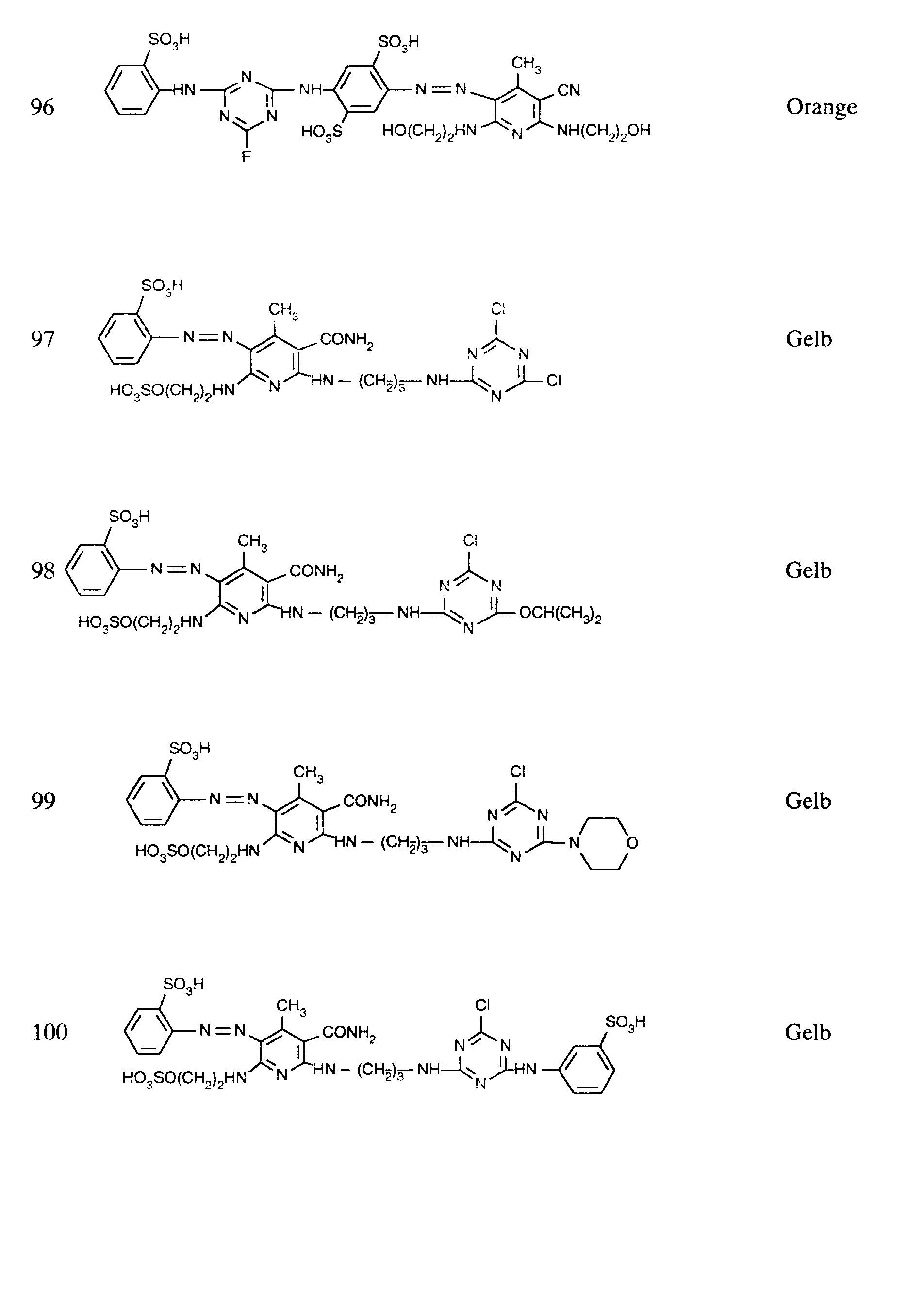

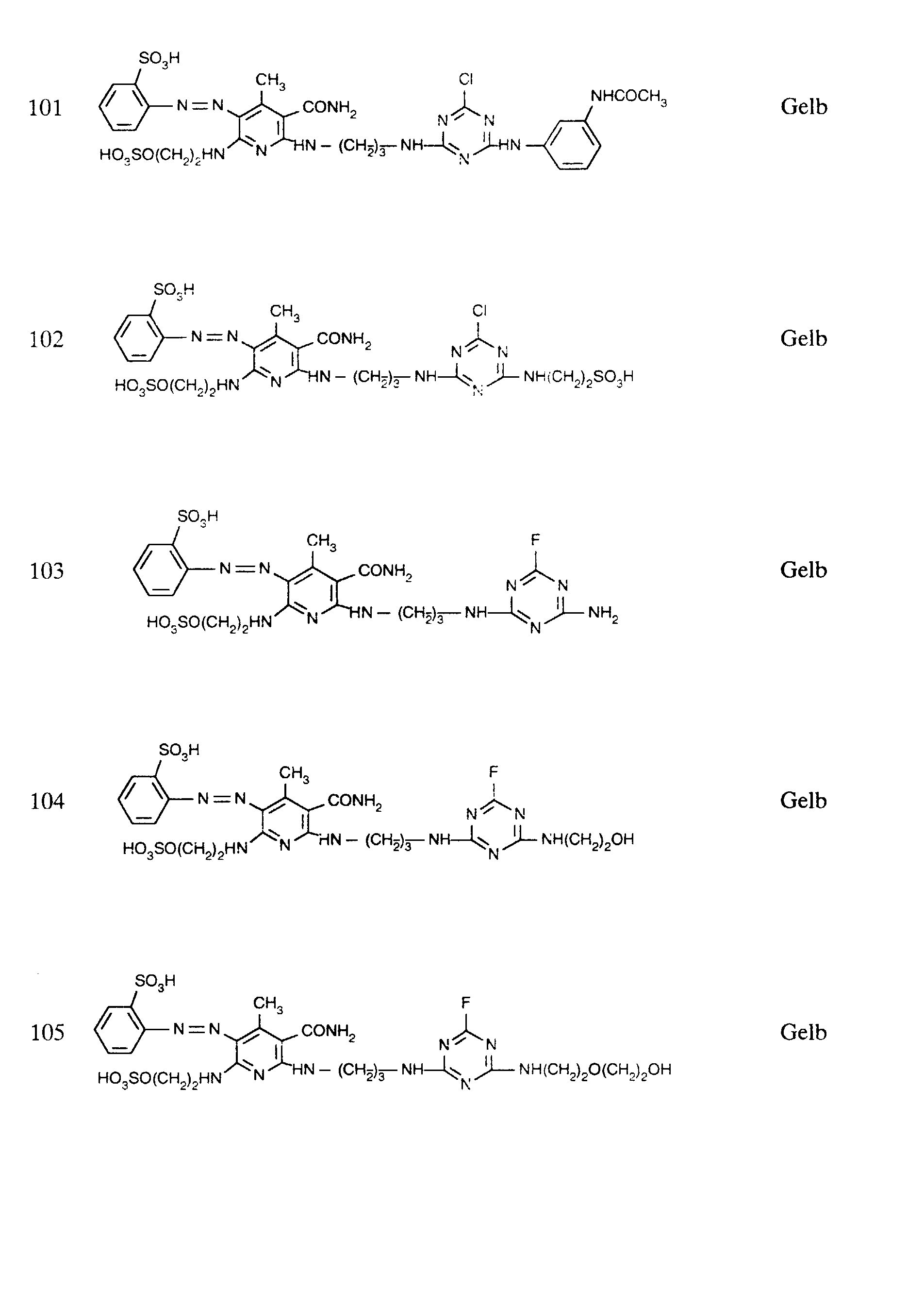

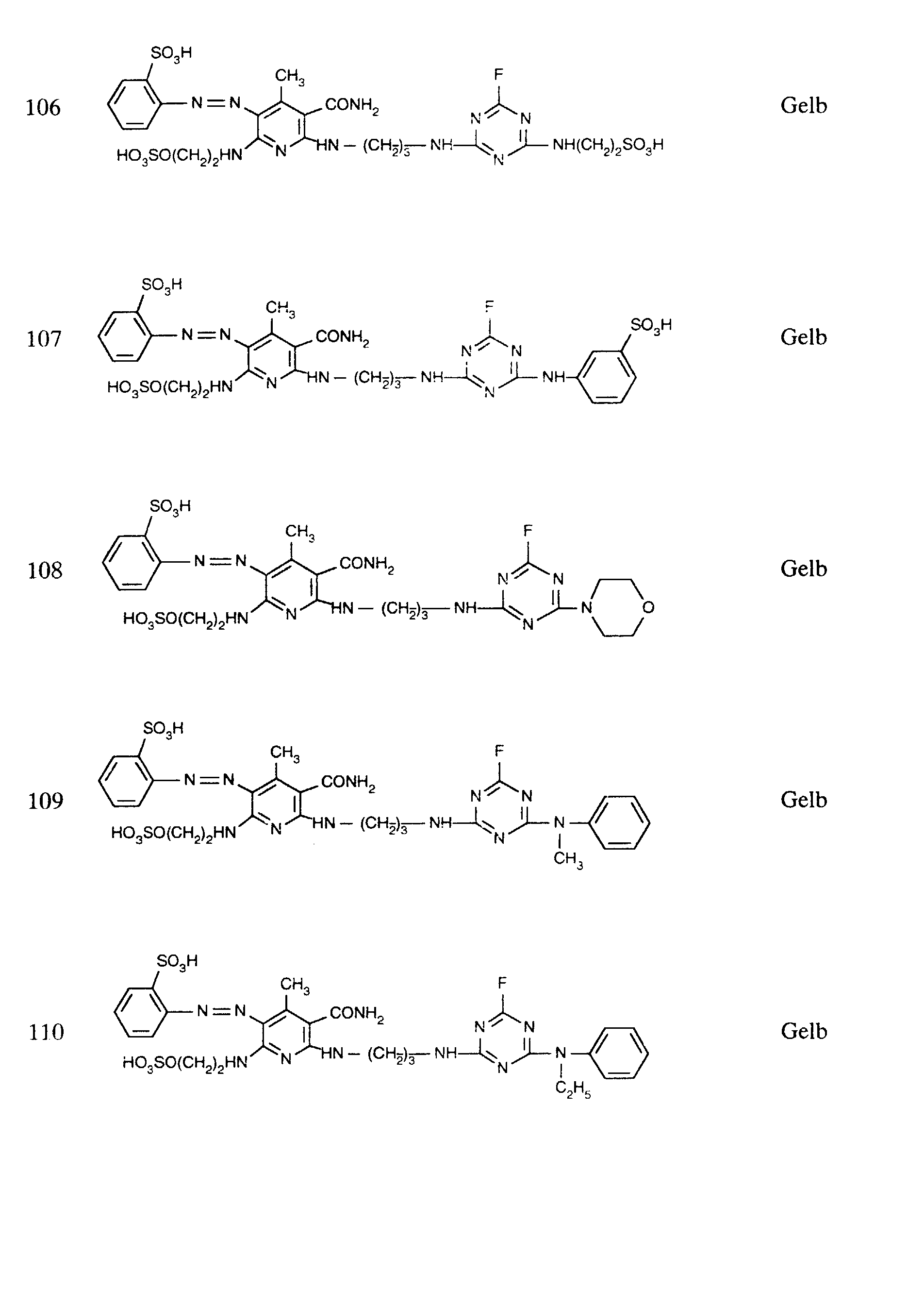

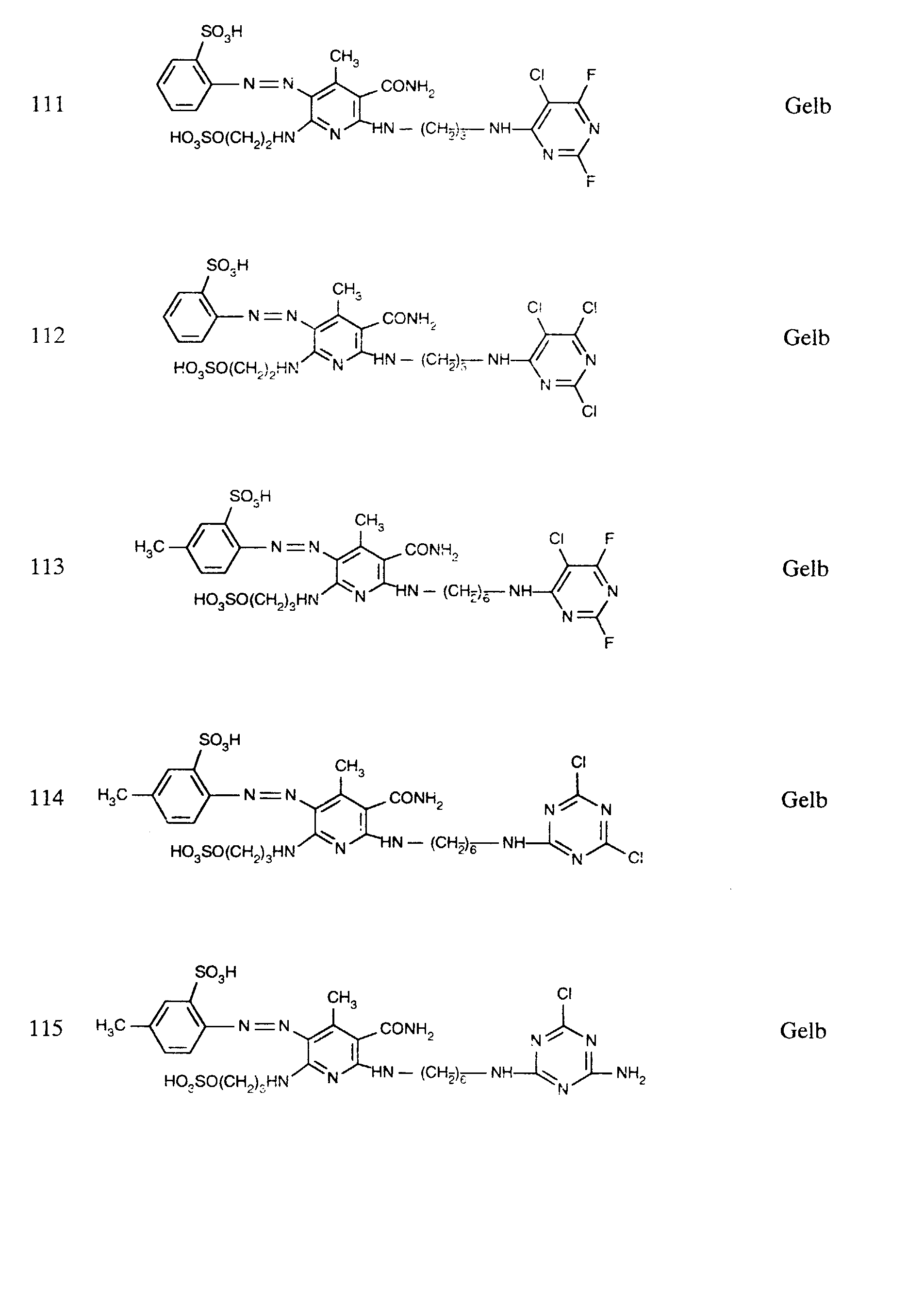

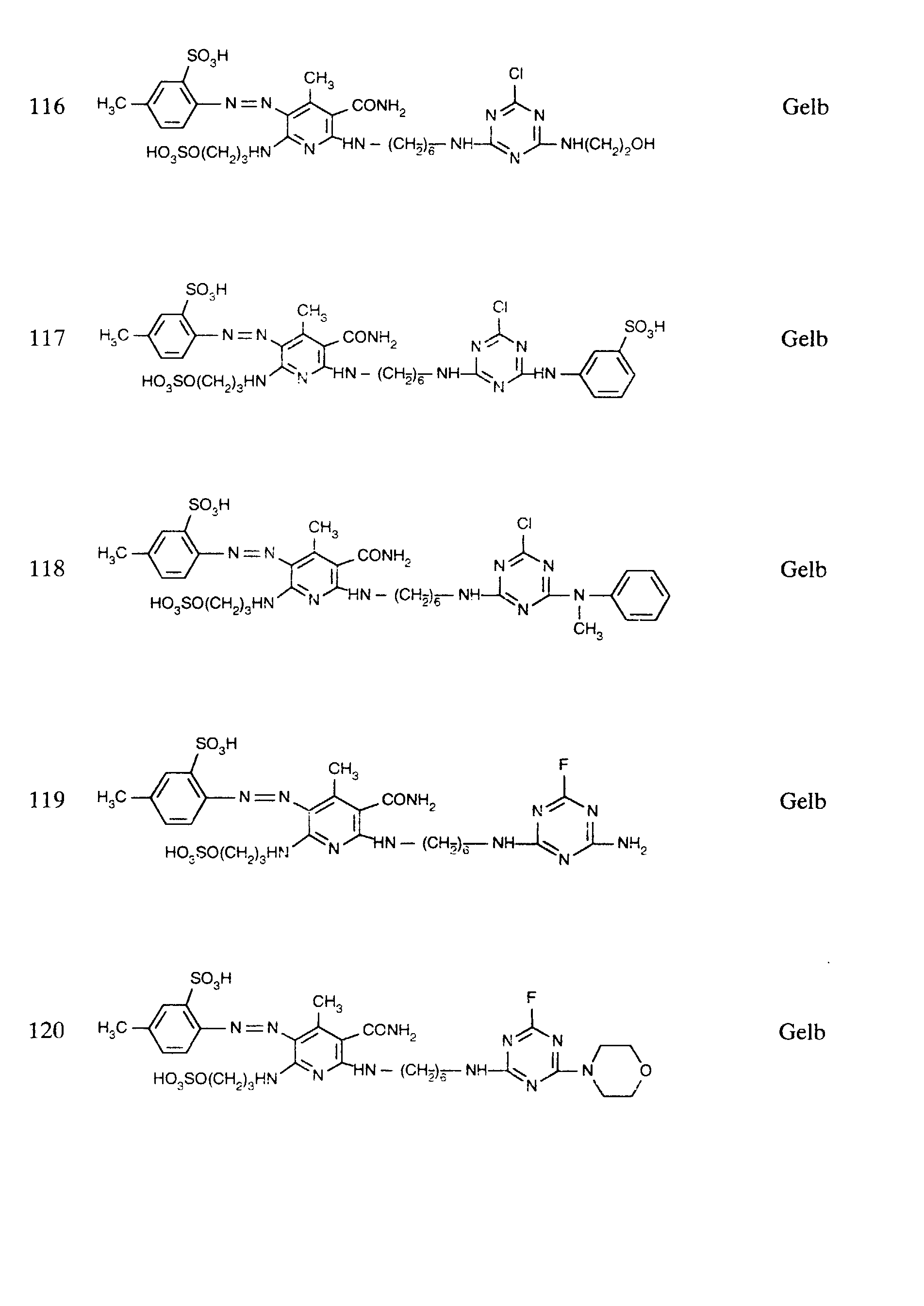

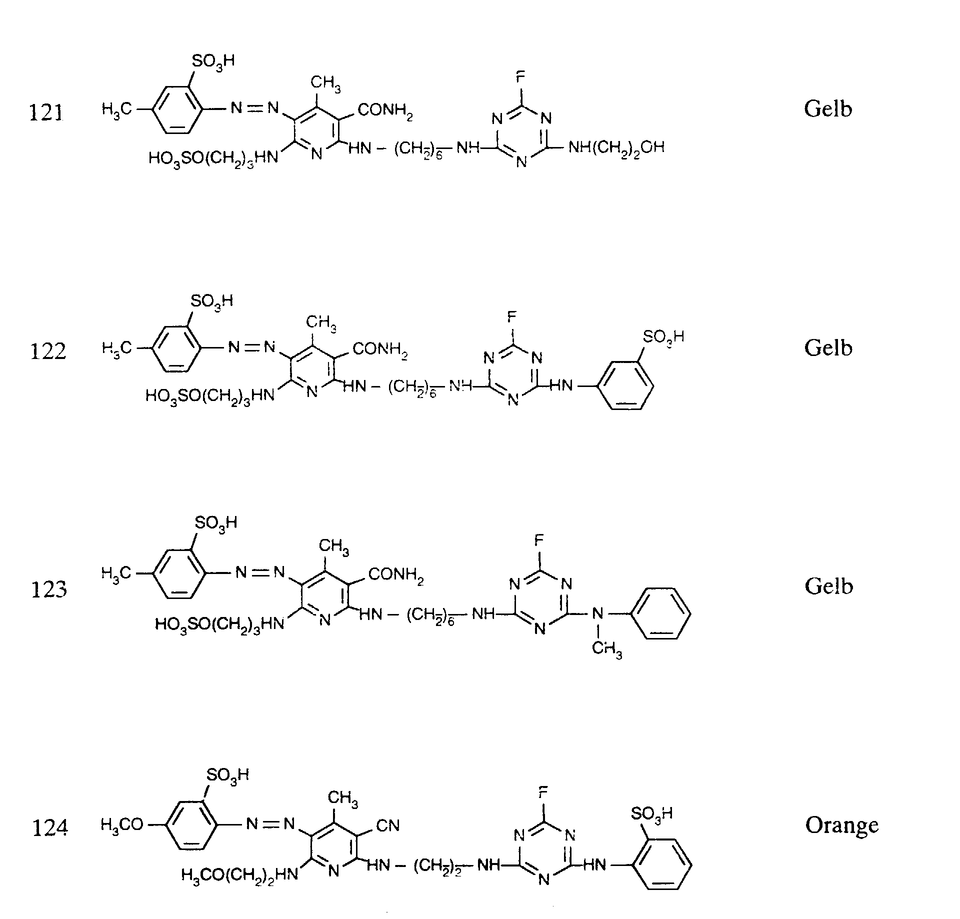

## Patentansprüche

1. Reaktivfarbstoffe der Formel 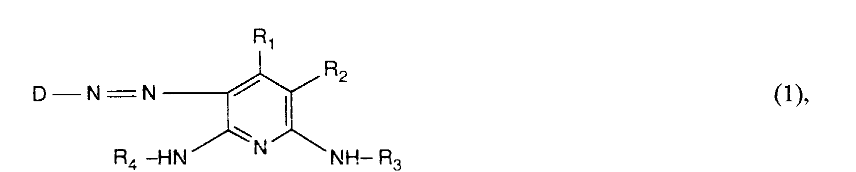 worin D der Rest einer Diazokomponente der Benzol- oder Naphthalinreihe oder der Rest eines Mono- oder Disazofarbstoffes ist,
R₁ C₁-C₄-Alkyl,
R₂ Cyano, Carbamoyl oder Sulfomethyl ist und
R₃ und R₄ unabhängig voneinander Wasserstoff, gegebenenfalls durch Hydroxyl, Sulfo oder Sulfato substituiertes und mit Ausnahme von Methyl gegebenenfalls durch Sauerstoff unterbrochenes C₁-C₁₂-Alkyl,
oder einen Rest der Formel 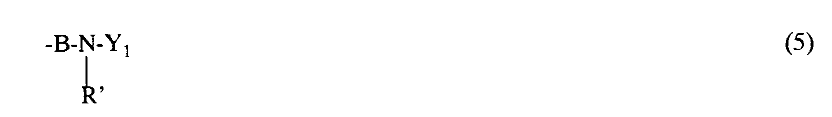 bedeuten, worin
R' Wasserstoff oder unsubstituiertes oder durch Hydroxyl, Sulfo oder Sulfato substituiertes und mit Ausnahme von Methyl gegebenenfalls durch Sauerstoff unterbrochenes C₁-C₁₂-Alkyl ist oder der Rest -(NR')- einen bivalenten 5- bis 7-gliedrigen aliphatischen Heterocyclus darstellt,
B gegebenenfalls durch Hydroxyl, Sulfo oder Sulfato substituiertes, und gegebenenfalls durch Sauerstoff unterbrochenes C₂-C₁₂-Alkylen und
Y₁ ein faserreaktiver Rest der Halogentriazin- oder Halogenpyrimidinreihe ist, und
der Reaktivfarbstoff der Formel (1) als faserreaktiven Rest einen Rest der Halogentriazinreihe, der Halogenpyrimidinreihe oder einen Rest der Formel 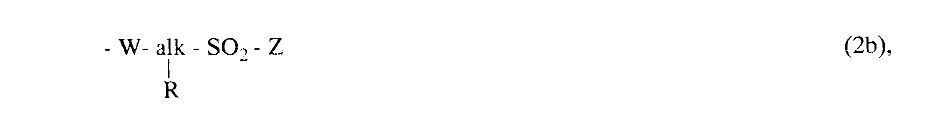
-W-alk-E-alk'-SO₂-Z (2c), 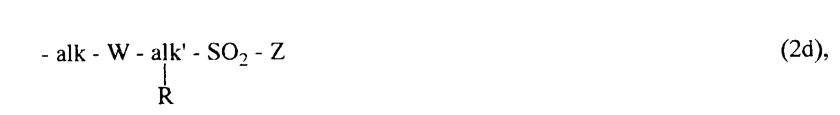 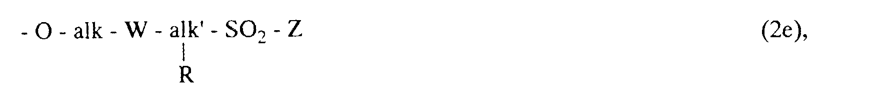 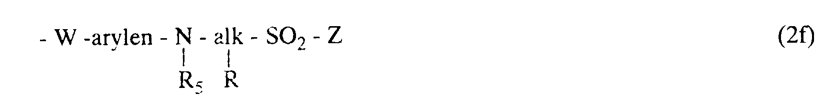
oder
-NH-CO-Z₁ (2g),
worin
W eine Gruppe der Formel -SO₂-NR₅-, -CONR₅- oder -NR₅CO- ist,
R₅ Wasserstoff, unsubstituiertes oder durch Hydroxy, Sulfo, Sulfato, Carboxy oder Cyano substituiertes C₁-C₄-Alkyl,
R Wasserstoff, Hydroxy, Sulfo, Sulfato, Carboxy, Cyano, Halogen, C₁-C₄-Alkoxycarbonyl, C₁-C₄-Alkanoyloxy oder Carbamoyl ist,
Z eine Gruppe der Formel -CH=CH₂ oder -CH₂-CH₂-U₁ und U₁ eine Abgangsgruppe ist,
Z₁ eine Gruppe der Formel 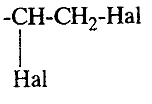 oder 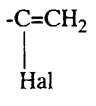 und Hal Halogen bedeutet,
E der Rest -O- oder -NR₆- und R₆ Wasserstoff oder C₁-C₄-Alkyl ist,
alk und alk' unabhängig voneinander C₁-C₆-Alkylen bedeuten und
arylen einen unsubstituierten oder durch Sulfo, Carboxy, C₁-C₄-Alkyl, C₁-C₄-Alkoxy oder Halogen substituierten Phenylen- oder Naphthylenrest bedeutet, enthält,
wobei der Reaktivfarbstoff der Formel (1) mindestens eine permanente Sulfo oder Sulfatogruppe und nur einen faserreaktiven Rest enthält.

2. Reaktivfarbstoffe gemäss Anspruch 1, dadurch gekennzeichnet, dass sie als faserreaktiven Rest der Halogentriazinreihe oder der Halogenpyrimidinreihe einen faserreaktiven Rest der Formel 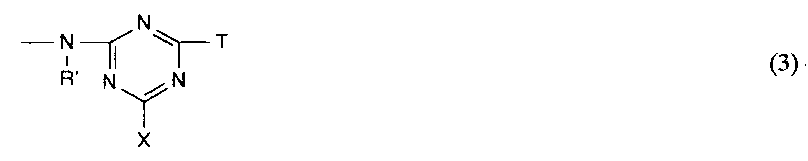 oder 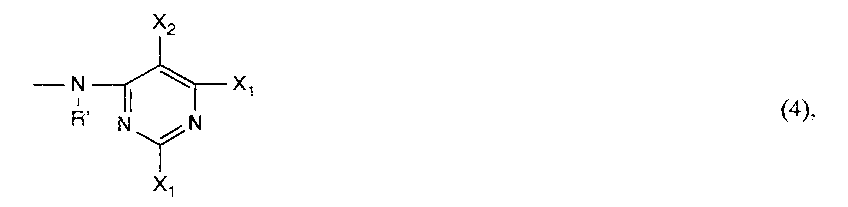 worin R' Wasserstoff oder unsubstituiertes oder durch Hydroxyl, Sulfo oder Sulfato substituiertes und mit Ausnahme von Methyl gegebenenfalls durch Sauerstoff unterbrochenes C₁-C₁₂-Alkyl ist oder der Rest -(NR')- einen bivalenten 5- bis 7-gliedrigen aliphatischen Heterocyclus darstellt, X, X₁ und X₂ Halogen bedeuten und T Halogen, Hydroxy, Sulfo, C₁-C₄-Alkoxy, C₁-C₄-Alkylthio, C₁-C₄-Alkylsulfonyl, Phenylsulfonyl, Morpholino oder gegebenenfalls substituiertes Amino ist, enthalten.

3. Reaktivfarbstoffe gemäss einem der Ansprüche 1 und 2, dadurch gekennzeichnet, dass R₃ und R₄ unabhängig voneinander Wasserstoff, gegebenenfalls durch Hydroxyl, Sulfo oder Sulfato substituiertes und mit Ausnahme von Methyl gegebenenfalls durch Sauerstoff unterbrochenes C₁-C₁₂-Alkyl,
oder einen Rest der Formel 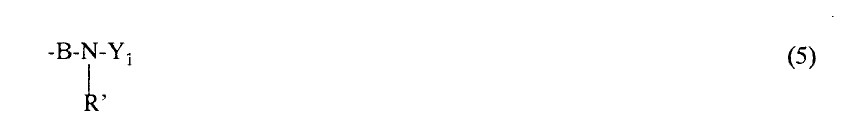 bedeuten, worin R' Wasserstoff oder unsubstituiertes oder durch Hydroxyl, Sulfo oder Sulfato substituiertes und mit Ausnahme von Methyl gegebenenfalls durch Sauerstoff unterbrochenes C₁-C₁₂-Alkyl ist oder der Rest -(NR')- einen bivalenten 5- bis 7-gliedrigen aliphatischen Heterocyclus darstellt, B gegebenenfalls durch Hydroxyl, Sulfo oder Sulfato substituiertes und gegebenenfalls durch Sauerstoff unterbrochenes C₂-C₁₂-Alkylen und Y₁ ein Reaktivrest der Formel 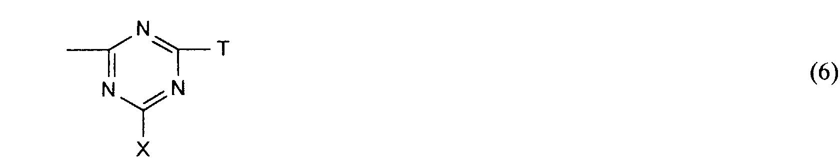 oder 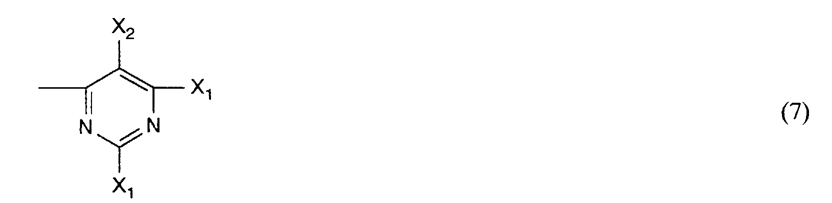 ist, wobei T, X, X₁ und X₂ die in Anspruch 2 angegebenen Bedeutungen haben.

4. Reaktivfarbstoffe gemäss einem der Ansprüche 1 bis 3, der Formel 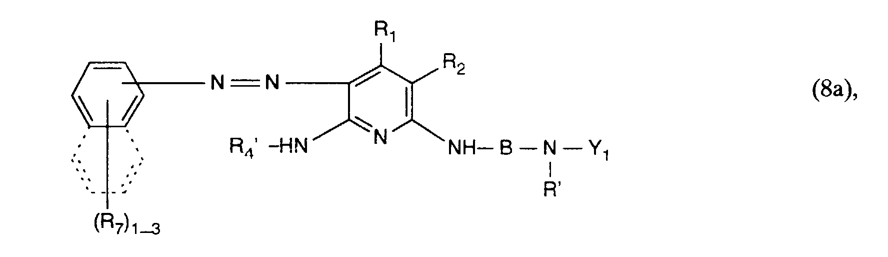 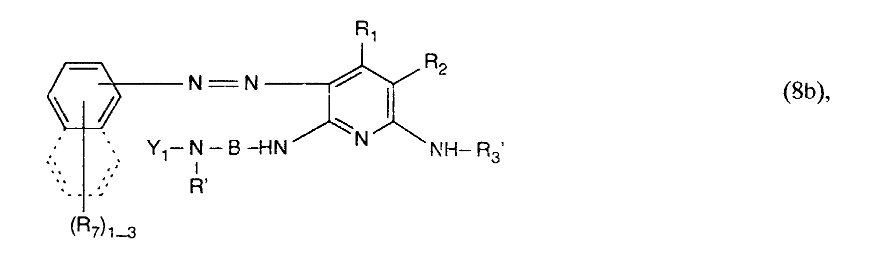 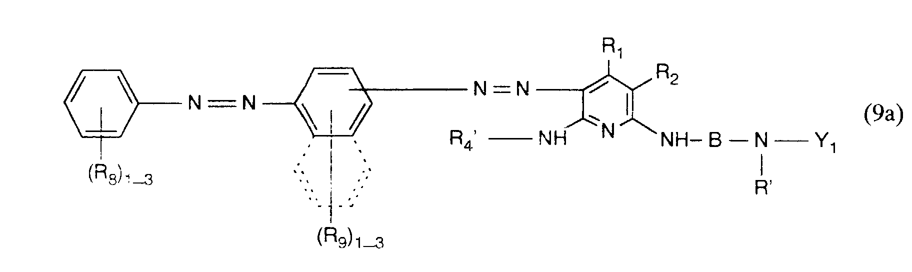 oder 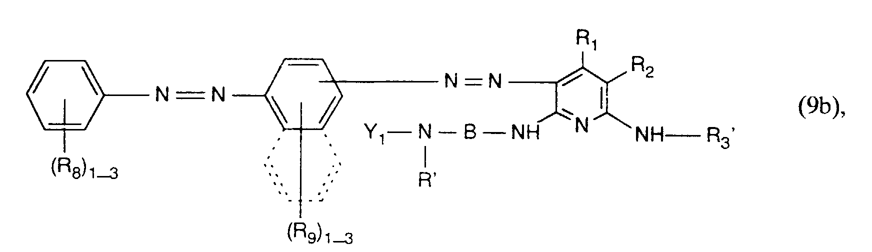 worin R₁ C₁-C₄-Alkyl, R₂ Cyano, Carbamoyl oder Sulfomethyl ist, R', R₃' und R₄' unabhängig voneinander Wasserstoff oder gegebenenfalls durch Hydroxyl, Sulfo oder Sulfato substituiertes und mit Ausnahme von Methyl gegebenenfalls durch Sauerstoff unterbrochenes C₁-C₁₂-Alkyl bedeuten, der Rest -(NR')- einen bivalenten 5- bis 7-gliedrigen aliphatischen Heterocyclus darstellen kann, (R₇)₁₋₃, (R₈)₁₋₃ und (R₉)₁₋₃ jeweils für 1 bis 3 gleiche oder verschiedene Substituenten aus der Gruppe Wasserstoff, C₁-C₄-Alkyl, C₁-C₄-Alkoxy, C₂-C₄-Alkanoylamino, Halogen und Sulfo stehen, B gegebenenfalls durch Hydroxyl, Sulfo oder Sulfato substituiertes und gegebenenfalls durch Sauerstoff unterbrochenes C₂-C₁₂-Alkylen und Y₁ ein faserreaktiver Rest der Formel (6) oder (7) gemäss Anspruch 3 ist.

5. Reaktivfarbstoffe gemäss einem der Ansprüche 1 bis 3, der Formel 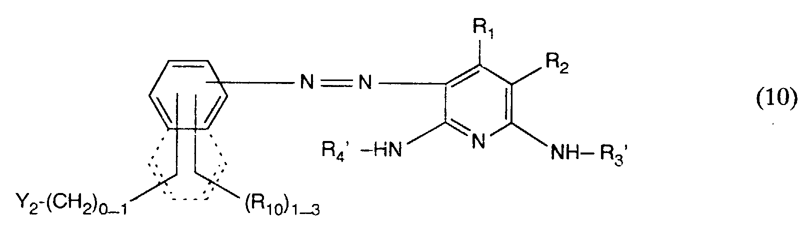 oder 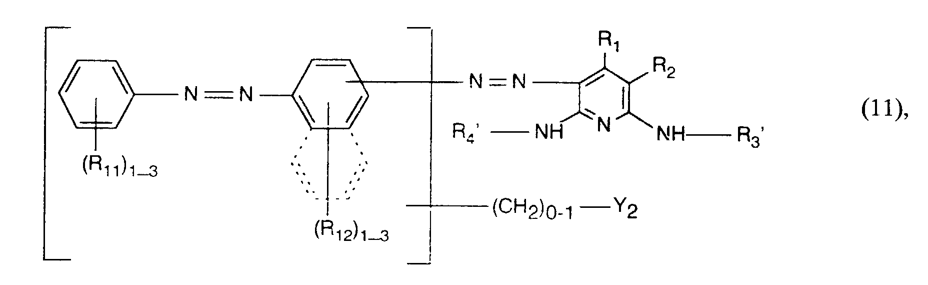 worin R₁ C₁-C₄-Alkyl, R₂ Cyano, Carbamoyl oder Sulfomethyl ist, R₃, und R₄' unabhängig voneinander Wasserstoff oder gegebenenfalls durch Hydroxyl, Sulfo oder Sulfato substituiertes und mit Ausnahme von Methyl gegebenenfalls durch Sauerstoff unterbrochenes C₁-C₁₂-Alkyl bedeuten, (R₁₀)₁₋₃, (R₁₁)₁₋₃ und (R₁₂)₁₋₃ jeweils für 1 bis 3 gleiche oder verschiedene Substituenten aus der Gruppe Wasserstoff, C₁-C₄-Alkyl, C₁-C₄-Alkoxy, C₂-C₄-Alkanoylamino, Halogen und Sulfo stehen und Y₂ ein faserreaktiver Rest der Formeln (2b) bis (2g), (3) oder (4) gemäss Anspruch 2 ist.

6. Reaktivfarbstoffe gemäss einem der Ansprüche 1 bis 5, dadurch gekennzeichnet, dass R₁ Methyl ist.

7. Reaktivfarbstoffe gemäss einem der Ansprüche 1 bis 6, dadurch gekennzeichnet, dass R₂ Cyano oder Carbamoyl ist.

8. Reaktivfarbstoffe gemäss einem der Ansprüche 1 bis 7, dadurch gekennzeichnet, dass B C₂-C₆-Alkylen bedeutet.

9. Reaktivfarbstoffe gemäss einem der Ansprüche 1 bis 8, dadurch gekennzeichnet, dass es sich bei dem Rest -(NR')- als bivalenten 5- bis 7-gliedrigen aliphatischen Heterocyclus um einen Rest der Formel 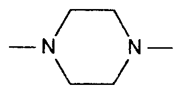 handelt.

10. Mischungen von Reaktivfarbstoffen, dadurch gekennzeichnet, dass sie mindestens zwei Reaktivfarbstoffe der Formel (1) gemäss Anspruch 1 enthalten.

11. Verfahren zur Herstellung von Reaktivfarbstoffen gemäss Anspruch 1, dadurch gekennzeichnet, dass man ein Amin der Formel
D-NH₂ (12),
worin D die in Anspruch 1 angegebenen Bedeutungen hat, diazotiert und auf eine Kupplungskomponente der Formel 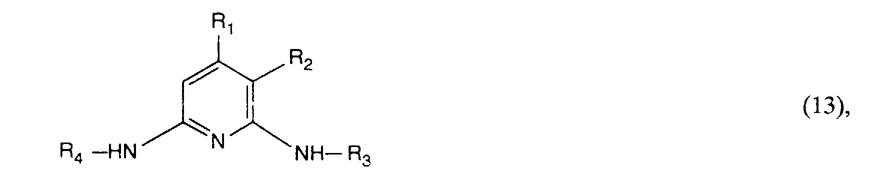 worin R₁, R₂, R₃ und R₄ die in Anspruch 1 angegebenen Bedeutungen haben, kuppelt, und gegebenenfalls eine weitere Umwandlungsreaktion anschliesst.

12. Verfahren zur Herstellung von Reaktivfarbstoffen gemäss Anspruch 1, dadurch gekennzeichnet, dass man eine Verbindung der Formel 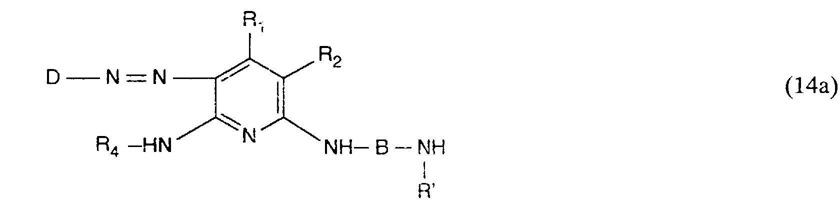 oder 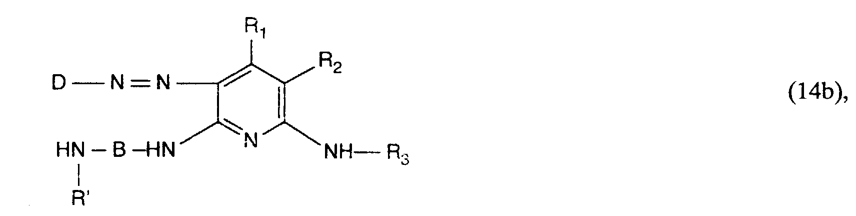 worin R', B, D, R₁, R₂, R₃ und R₄ die in Anspruch 1 angegebenen Bedeutungen haben, wobei die Verbindungen der Formeln (14a) und (14b) keinen faserreaktiven Rest enthalten, mit einem faserreaktiven Rest der Halogentriazin- oder Halogenpyrimidinreihe kondensiert.

13. Verwendung der Reaktivfarbstoffe gemäss einem der Ansprüche 1 bis 9 bzw. der Farbstoffmischungen gemäss Anspruch 10 zum Färben oder Bedrucken von hydroxylgruppenhaltigen oder stickstoffhaltigen Fasermaterialien.

14. Verwendung gemäss Anspruch 13 zum Färben oder Bedrucken von cellulosehaltigen Fasermaterialien oder natürlichen oder synthetischen Polyamidfasermaterialien.

## Claims

1. A reactive dye of the formula 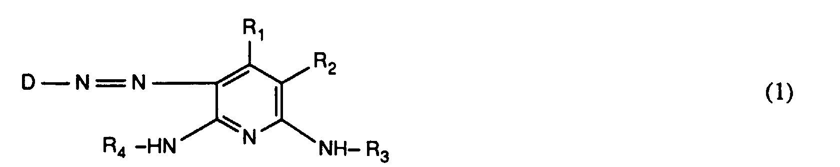 in which D is the radical of a diazo component of the benzene or naphthalene series or the radical of a mono- or disazo dye,
R₁ is C₁-C₄alkyl,
R₂ is cyano, carbamoyl or sulfomethyl and
R₃ and R₄ independently of one another are hydrogen, C₁-C₁₂alkyl which is unsubstituted or substituted by hydroxyl, sulfo or sulfato and, with the exception of methyl, may be interrupted by oxygen,
or a radical of the formula 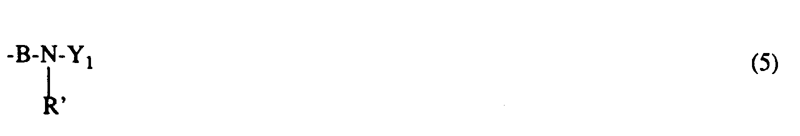 in which
R' is hydrogen or C₁-C₁₂alkyl which is unsubstituted or substituted by hydroxyl, sulfo or sulfato and, with the exception of methyl, may be interrupted by oxygen, or the radical -(NR')- is a divalent 5- to 7-membered aliphatic heterocyclic radical,
B is C₂-C₁₂alkylene which is unsubstituted or substituted by hydroxyl, sulfo or sulfato and may be interrupted by oxygen and
Y₁ is a fibre-reactive radical of the halotriazine or halopyrimidine series, and
the reactive dye of the formula (1) contains, as the fibre-reactive radical, a radical of the halotriazine series or halopyrimidine series or a radical of the formula 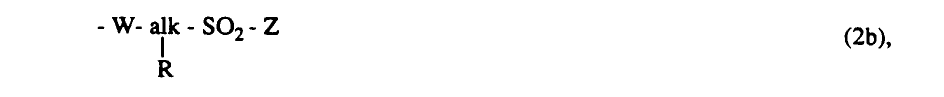
-W-alk-E-alk'-SO₂Z (2c), 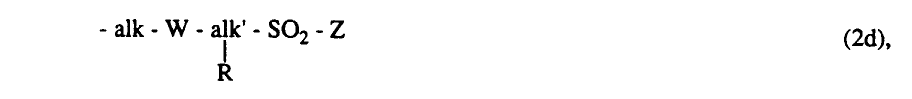 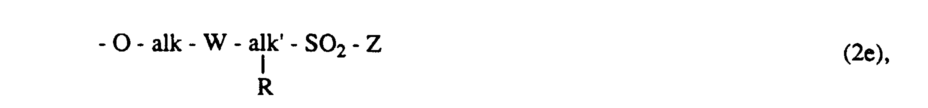 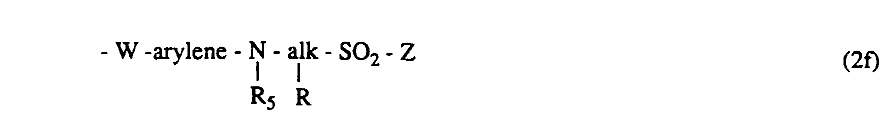
or
-NH-CO-Z₁ (2g)
in which
W is a group of the formula -SO₂-NR₅-, -CONR₅ or -NR₅CO-,
R₅ is hydrogen, or C₁-C₄alkyl which is unsubstituted or substituted by hydroxyl, sulfo, sulfato, carboxyl or cyano,
R is hydrogen, hydroxyl, sulfo, sulfato, carboxyl, cyano, halogen, C₁-C₄alkoxycarbonyl, C₁-C₄alkanoyloxy or carbamoyl,
Z is a group of the formula -CH=CH₂ or -CH₂-CH₂-U₁ and U₁ is a leaving group,
Z₁ is a group of the formula 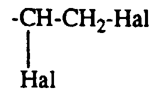 or 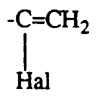 and Hal is halogen,
E is the radical -O- or -NR₆- and R₆ is hydrogen or C₁-C₄alkyl,
alk and alk' independently of one another are C₁-C₆alkylene and
arylene is a phenylene or naphthylene radical which is unsubstituted or substituted by sulfo, carboxyl, C₁-C₄alkyl, C₁-C₄alkoxy or halogen,
the reactive dye of the formula (1) containing at least one permanent sulfo or sulfato group and only one fibre-reactive radical.

2. A reactive dye according to claim 1, which contains as the fibre-reactive radical of the halotriazine series or of the halopyrimidine series a fibre-reactive radical of the formula 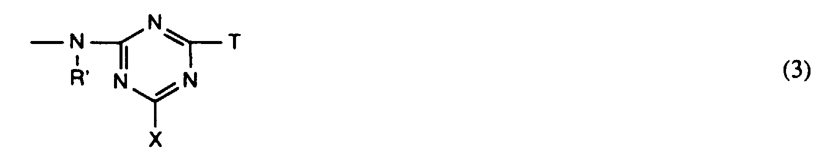 or 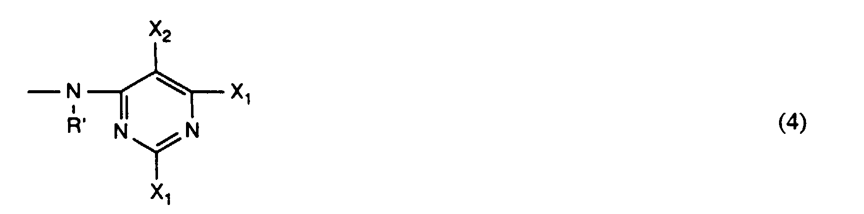 in which R' is hydrogen or C₁-C₁₂alkyl which is unsubstituted or substituted by hydroxyl, sulfo or sulfato and, with the exception of methyl, may be interrupted by oxygen, or the radical -(NR')- is a divalent 5- to 7-membered aliphatic heterocyclic radical, X, X₁ and X₂ are halogen and T is halogen, hydroxyl, sulfo, C₁-C₄alkoxy, C₁-C₄alkylthio, C₁-C₄alkylsulfonyl, phenylsulfonyl, morpholino or unsubstituted or substituted amino.

3. A reactive dye according to either of claims 1 and 2, in which R₃ and R₄ independently of one another are hydrogen, C₁-C₁₂alkyl which is unsubstituted or substituted by hydroxyl, sulfo or sulfato and, with the exception of methyl, may be interrupted by oxygen,
or a radical of the formula 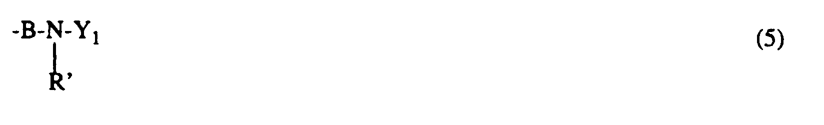 in which R' is hydrogen or C₁-C₁₂alkyl which is unsubstituted or substituted by hydroxyl, sulfo or sulfato and, with the exception of methyl, may be interrupted by oxygen, or the radical -(NR')- is a divalent 5- to 7-membered aliphatic heterocyclic radical, B is C₂-C₁₂alkylene which is unsubstituted or substituted by hydroxyl, sulfo or sulfato and may be interrupted by oxygen and Y₁ is a reactive radical of the formula 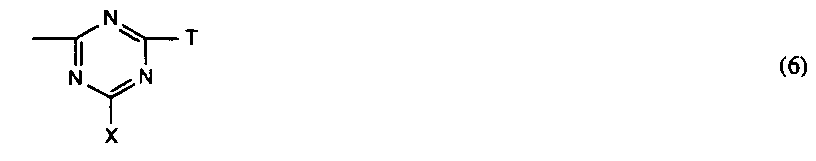 or 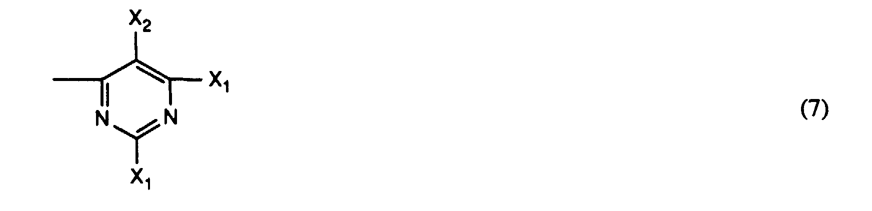 in which T, X, X₁ and X₂ are as defined in claim 2.

4. A reactive dye according to any one of claims 1 to 3, of the formula 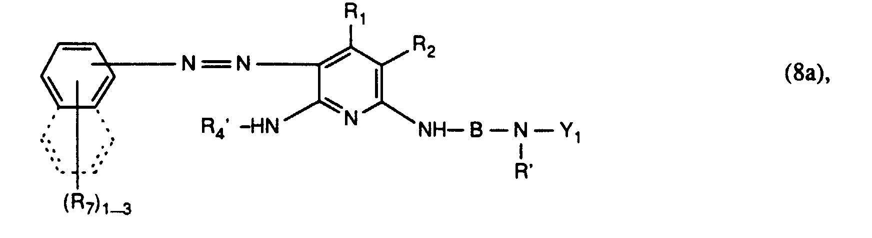 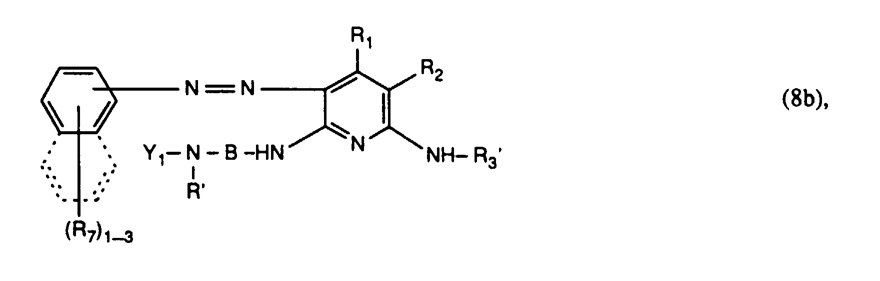 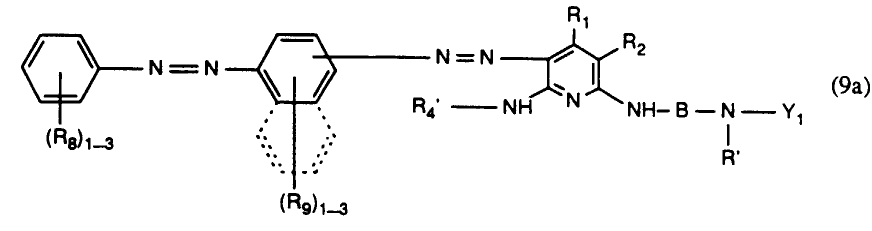 or 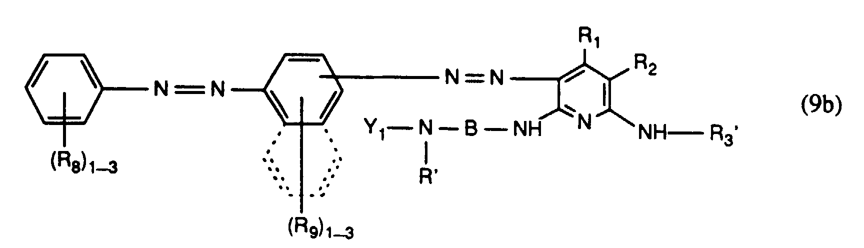 in which R₁ is C₁-C₄alkyl, R₂ is cyano, carbamoyl or sulfomethyl, R', R₃' and R₄' independently of one another are hydrogen or C₁-C₁₂alkyl which is unsubstituted or substituted by hydroxyl, sulfo or sulfato and, with the exception of methyl, may be interrupted by oxygen, the radical -(NR')- can be a divalent 5- to 7-membered aliphatic heterocyclic radical, (R₇)₁₋₃, (R₈)₁₋₃ and (R₉)₁₋₃ are in each case 1 to 3 identical or different substituents from the group comprising hydrogen, C₁-C₄alkyl, C₁-C₄alkoxy, C₂-C₄alkanoylamino, halogen and sulfo, B is C₂-C₁₂alkylene which is unsubstituted or substituted by hydroxyl, sulfo and sulfato and may be interrupted by oxygen and Y₁ is a fibre-reactive radical of the formula (6) or (7) according to claim 3.

5. A reactive dye according to any one of claims 1 to 3, of the formula 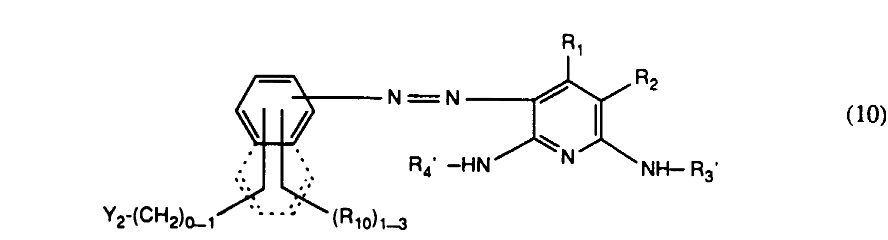 or 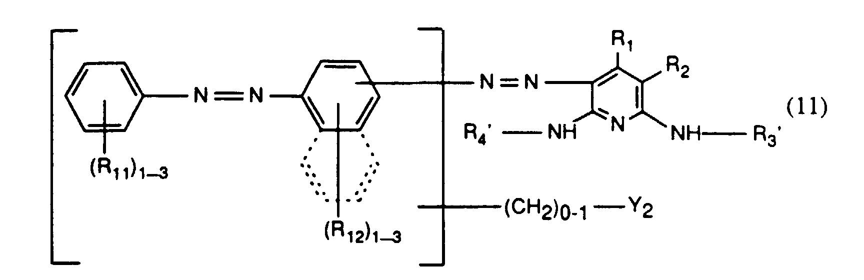 in which R₁ is C₁-C₄alkyl, R₂ is cyano, carbamoyl or sulfomethyl, R₃' and R₄' independently of one another are hydrogen or C₁-C₁₂alkyl which is unsubstituted or substituted by hydroxyl, sulfo or sulfato and, with the exception of methyl, may be interrupted by oxygen, (R₁₀)₁₋₃, (R₁₁)₁₋₃ and (R₁₂)₁₋₃ in each case are 1 to 3 identical or different substituents from the group comprising hydrogen, C₁-C₄alkyl, C₁-C₄alkoxy, C₂-C₄alkanoylamino, halogen and sulfo and Y₂ is a fibre-reactive radical of the formulae (2b) to (2g), (3) or (4) according to claim 2.

6. A reactive dye according to any one of claims 1 to 5, in which R₁ is methyl.

7. A reactive dye according to any one of claims 1 to 6, in which R₂ is cyano or carbamoyl.

8. A reactive dye according to any one of claims 1 to 7, in which B is C₂-C₆alkylene.

9. A reactive dye according to any one of claims 1 to 8, in which the divalent 5- to 7-membered aliphatic heterocyclic radical -(NR')- is a radical of the formula

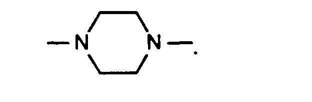

10. A mixture of reactive dyes which comprises at least two reactive dyes of the formula (1) according to claim 1.

11. A process for the preparation of a reactive dye according to claim 1, which comprises diazotizing an amine of the formula
D-NH₂ (12)
in which D is as defined in claim 1, and coupling the diazotization product to a coupling component of the formula 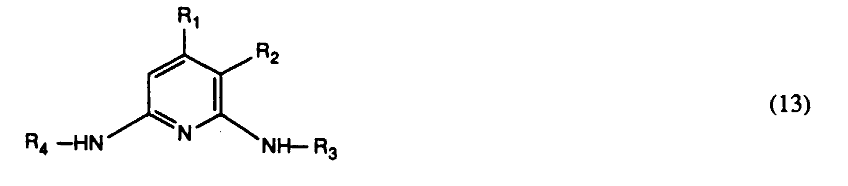 in which R₁, R₂, R₃ and R₄ are as defined in claim 1, and if appropriate subsequently carrying out a further conversion reaction.

12. A process for the preparation of a reactive dye according to claim 1, which comprises condensing a compound of the formula 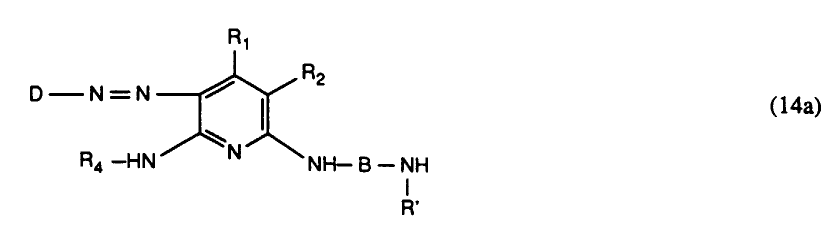 or 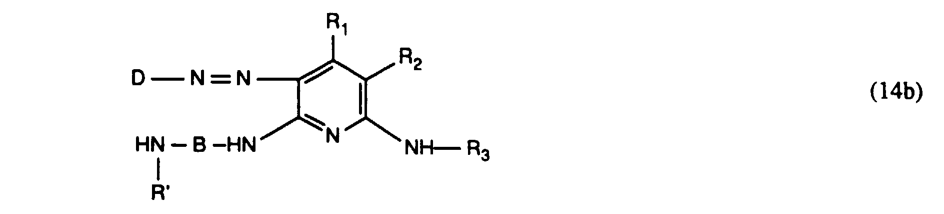 in which R', B, D, R₁, R₂, R₃ and R₄ are as defined in claim 1, the compounds of the formulae (14a) and (14b) containing no fibre-reactive radical, with a fibre-reactive radical of the halotriazine or halopyrimidine series.

13. The use of a reactive dye according to any one of claims 1 to 9 or a dye mixture according to claim 10 for dyeing or printing fibre materials containing hydroxyl groups or nitrogen.

14. The use according to claim 13 for dyeing or printing cellulosic fibre materials or naturally occurring or synthetic polyamide fibre materials.

## Revendications

1. Colorants réactifs de formule 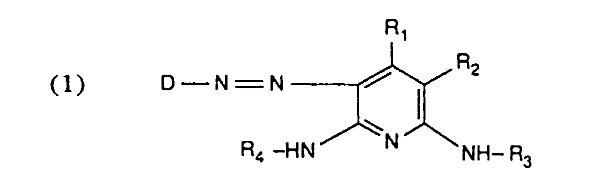 dans laquelle
D représente le résidu d'un composant diazo de la série benzénique ou naphtalénique ou le résidu d'un colorant monoazoïque ou diazoïque,
R₁ représente un résidu alkyle en C₁₋₄,
R₂ représente un résidu cyano, carbamoyle ou sulfométhyle, et
R₃ et R₄ représentent, indépendamment l'un de l'autre, un atome d'hydrogène, un résidu alkyle en C₁₋₁₂ portant éventuellement un ou plusieurs substituants hydroxyle, sulfo ou sulfato et dont la chaîne, sauf lorsqu'il s'agit d'un résidu méthyle, peut être interrompue par un ou plusieurs atomes d'oxygène, ou un résidu de formule 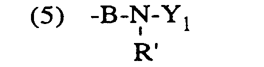 où R' représente un atome d'hydrogène ou un résidu alkyle en C₁₋₁₂ portant éventuellement un ou plusieurs substituants hydroxyle, sulfo ou sulfato, et dont la chaîne, sauf lorsqu'il s'agit d'un résidu méthyle, peut être interrompue par un ou plusieurs atomes d'oxygène, ou le résidu -(NR')-représente un hétérocycle aliphatique comportant de 5 à 7 chaînons,
B représente un groupe alkylène en C₂₋₁₂ portant éventuellement un ou plusieurs substituants hydroxyle, sulfo ou sulfato, et pouvant être interrompu par un ou plusieurs atomes d'oxygène, et
Y₁ représente un résidu réactif vis-à-vis des fibres de la série des halogénotriazines ou halogénopyrimidines,
et le colorant réactif de formule (1) contenant, comme résidu réactif vis-à-vis des fibres, un résidu de la série des halogénotriazines ou halogénopyrimidines, ou un résidu de formule 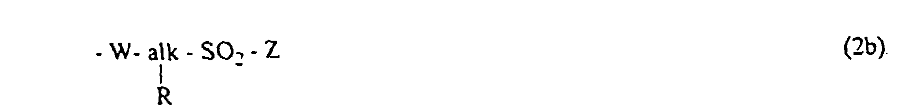
-W-alk-E-alk'-SO₂-Z (2c) 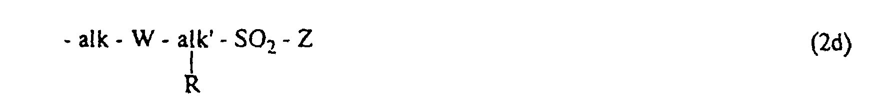 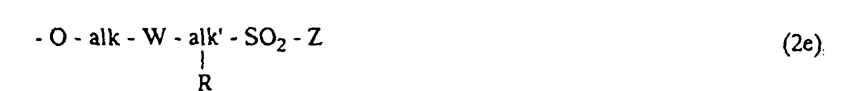 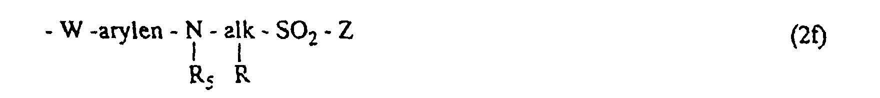
-NH-CO-Z₁ (2g)
où
W représente un groupe de formule -SO₂-NR₅-, -CONR₅- ou -NR₅CO-
R₅ représente un atome d'hydrogène, un groupe alkyle en C₁₋₄ non substitué ou portant un ou plusieurs substituants hydroxy, sulfo, sulfato, carboxy ou cyano,
R représente un atome d'hydrogène ou un groupe hydroxy, sulfo, sulfato, carboxy, cyano, halogéno, (alcoxy en C₁₋₄)-carbonyle, alcanoyloxy en C₁₋₄ ou carbamoyle,
Z représente un groupe de formule -CH=CH₂ ou -CH₂-CH₂-U₁ où U₁ est un groupe partant,
Z₁ représente un groupe de formule -CH(*Hal*)-CH₂-*Hal* ou -C(*Hal*)=CH₂ et *Hal* représente un atome d'halogène,
E est un résidu -O- ou -NR₆- et R₆ représente un atome d'hydrogène ou un groupe alkyle en C₁₋₄,
*alk* et *alk'* représentent indépendamment l'un de l'autre un groupe alkylène en C₁₋₆, et
*arylène* représente un groupe phénylène ou naphtylène non substitué ou portant un ou plusieurs résidus sulfo, carboxy, alkyle en C₁₋₄. alcoxy en C₁₋₄ ou halogéno ; le colorant réactif de formule (1) contenant au moins un groupe sulfo ou sulfato permanent et un seul résidu réactif vis-à-vis des fibres.

2. Colorants réactifs selon la revendication 1, caractérisés en ce qu'ils contiennent comme résidu réactif vis-à-vis des fibres de la série des halogénotriazines ou de la série des halogénopyrimidines un résidu réactif vis-à-vis des fibres de formule 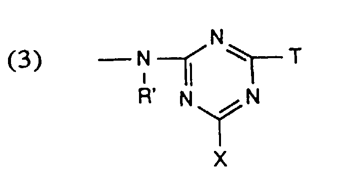 ou 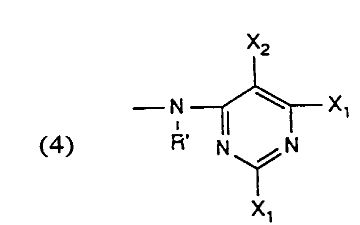 dans lesquelles
R' représente un atome d'hydrogène ou un résidu alkyle en C₁₋₁₂ éventuellement substitué et pouvant contenir un atome d'oxygène dans la chaîne, sauf lorsqu'il s'agit d'un groupe méthyle, ou le résidu -(NR')-représente un hétérocycle aliphatique bivalent à 5 ou 7 chaînons,
X, X₁ et X₂ représentent un atome d'halogène,
T représente un atome d'halogène ou un groupe hydroxy, sulfo, alcoxy en C₁₋₄, alkylthio en C₁₋₄, alkylsulfonyle en C₁₋₄, phénylsulfonyle, morpholino ou amino éventuellement substitué.

3. Colorants réactifs selon une des revendications 1 et 2, caractérisés en ce que R₃ et R₄ représentent, indépendamment l'un de l'autre, un atome d'hydrogène ou un groupe alkyle en C₁₋₁₂ non substitué ou portant un ou plusieurs substituants hydroxyle, sulfo ou sulfato et pouvant être interrompu, sauf lorsqu'il s'agit d'un groupe méthyle, par un ou plusieurs atomes d'oxygène, ou un résidu de formule 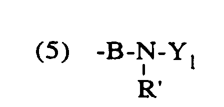 dans laquelle R' représente un atome d'hydrogène ou un groupe alkyle en C₁₋₁₂ non substitué ou portant un ou plusieurs substituants hydroxyle, sulfo ou sulfato et pouvant être interrompu, sauf lorsqu'il s'agit d'un groupe méthyle, par un ou plusieurs atomes d'oxygène, ou le résidu -(NR')- représente un hétérocycle aliphatique comportant de 5 à 7 chaînons, B représente un groupe alkylène en C₂₋₁₂ non substitué ou portant un ou plusieurs substituants hydroxyle, sulfo ou sulfato et pouvant être interrompu par un ou plusieurs atomes d'oxygène, et Y₁ représente un résidu réactif de formule 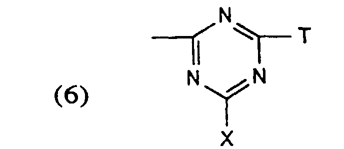 ou 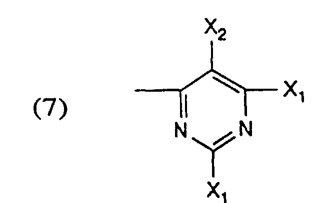 où T, X, X₁ et X₂ ont les significations indiquées dans la revendication 2.

4. Colorants réactifs selon l'une quelconque des revendications 1 à 3, de formule 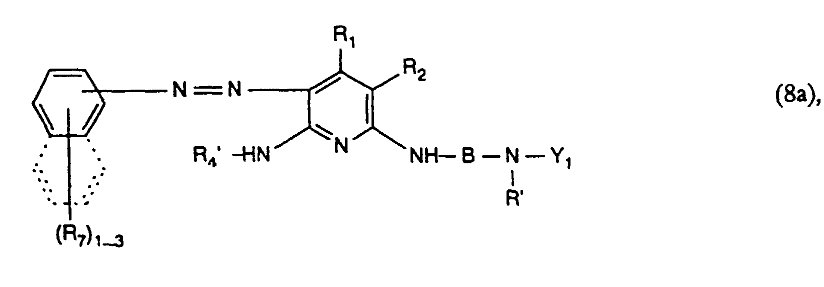 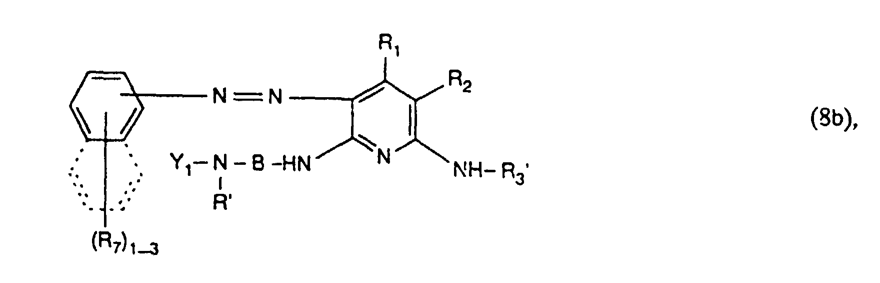 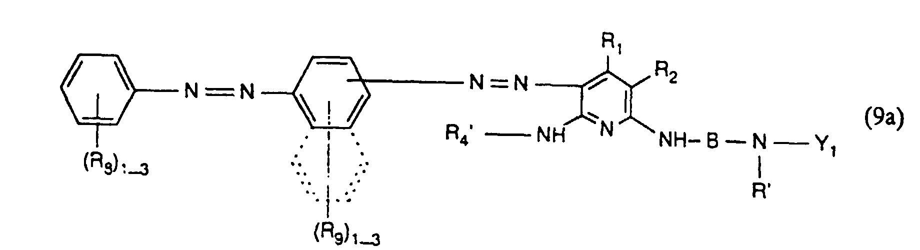 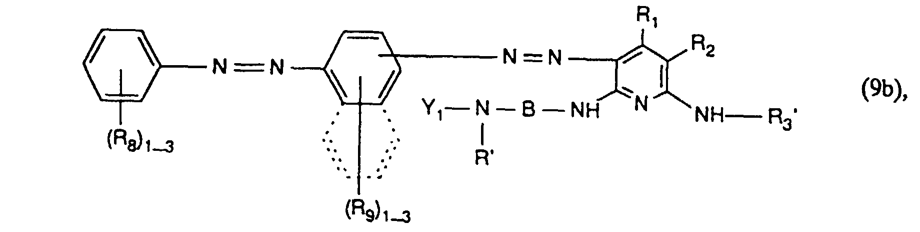 dans lesquelles R₁ représente un groupe alkyle en C₁₋₄, R₂ représente un groupe cyano, carbamoyle ou sulfométhyle, R', R₃' et R₄' représentent indépendamment un atome d'hydrogène ou un groupe alkyle en C₁₋₁₂ non substitué ou portant un ou plusieurs substituants hydroxyle, sulfo ou sulfato et pouvant être interrompu, sauf lorsqu'il s'agit d'un groupe méthyle, par un ou plusieurs atomes d'oxygène, le résidu -(NR')- peut représenter un hétérocycle aliphatique bivalent comportant de 5 à 7 chaînons, (R₇)₁₋₃, (R₈)₁₋₃ et (R₉)₁₋₃ représentent chacun de 1 à 3 substituants identiques ou différents choisis dans le groupe formé par les résidus hydrogéno, alkyle en C₁₋₄, alcoxy en C₁₋₄, alcanoylamino en C₂₋₄, halogéno et sulfo, B représente un groupe alkylène en C₂₋₁₂ non substitué ou portant un ou plusieurs substituants hydroxyle, sulfo ou sulfato et pouvant être interrompu par un ou plusieurs atomes d'oxygène, et Y₁ est un résidu réactif vis-à-vis des fibres de formule (6) ou (7) selon la revendication 3.

5. Colorants réactifs selon l'une quelconque des revendications 1 à 3, de formule 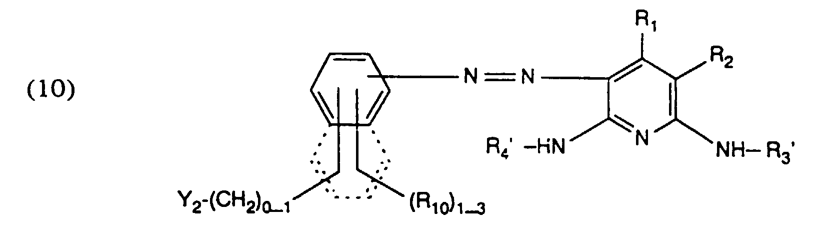 ou 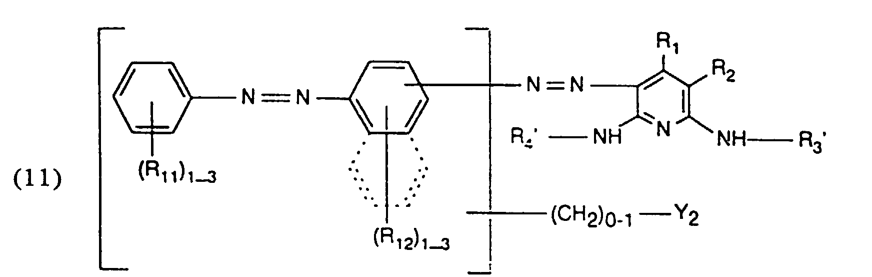 dans lesquelles R₁ représente un groupe alkyle en C₁₋₄, R₂ représente un groupe cyano, carbamoyle ou sulfométhyle, R₃' et R₄' représentent indépendamment un atome d'hydrogène ou un groupe alkyle en C₁₋₁₂ non substitué ou portant un ou plusieurs substituants hydroxyle, sulfo ou sulfato et pouvant être interrompu, sauf lorsqu'il s'agit d'un groupe méthyle, par un ou plusieurs atomes d'oxygène, (R₁₀)₁₋₃, (R₁₁)₁₋₃ et (R₁₂)₁₋₃ représentent chacun de 1 à 3 substituants identiques ou différents choisis dans le groupe formé par un les résidus hydrogéno, alkyle en C₁₋₄, alcoxy en C₁₋₄, alcanoylamino en C₂₋₄, halogéno ou sulfo et Y₂ est un résidu réactif vis-à-vis des fibres de formule (2b) à (2g), (3) ou (4) selon la revendication 2.

6. Colorants réactifs selon une des revendications 1 à 5, caractérisés en ce que R₁ représente un groupe méthyle.

7. Colorants réactifs selon une des revendications 1 à 6, caractérisés en ce que R₂ représente un résidu cyano ou carbamoyle.

8. Colorants réactifs selon une des revendications 1 à 7, caractérisés en ce que B représente un groupe alkylène en C₂₋₆.

9. Colorants réactifs selon une des revendications 1 à 8, caractérisés en ce que le résidu -(NR')-, lorsqu'il s'agit d'un hétérocycle aliphatique bivalent comportant de 5 à 7 chaînons, est un résidu de formule

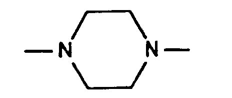

10. Mélanges de colorants réactifs, caractérisés en ce qu'ils contiennent au moins deux colorants réactifs de formule (1) selon la revendication 1.

11. Procédé de préparation de colorants réactifs selon la revendication 1, caractérisé en ce que l'on procède à la diazotation d'une amine de formule
(12) D-NH₂
où D a la signification indiquée dans la revendication 1, pour la coupler ensuite avec un copulant de formule 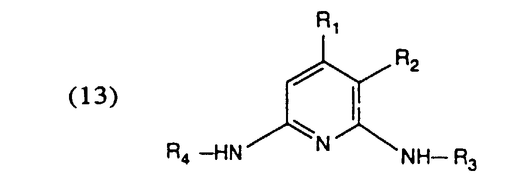 dans laquelle R₁, R₂, R₃ et R₄ ont les significations indiquées dans la revendication 1, et que l'on fait suivre éventuellement une réaction de conversion supplémentaire.

12. Procédé de préparation de colorants réactifs selon la revendication 1, caractérisé en ce que l'on effectue une réaction de condensation entre
- un composé de formule 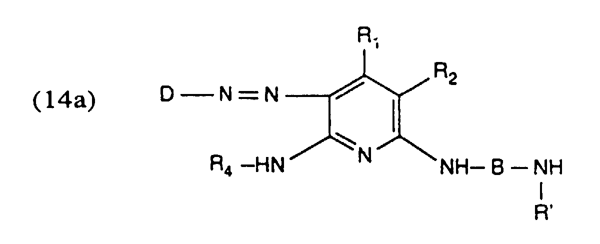 ou 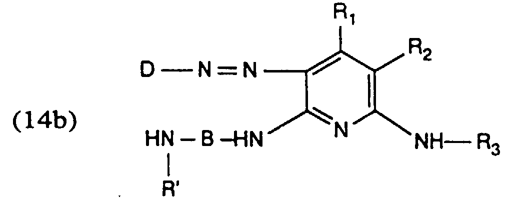 dans lesquelles R', B, D, R₁, R₂, R₃ et R₄ ont les significations indiquées dans la revendication 1, lesquels composés de formule (14a) et (14b) ne contenant pas de résidus réactifs vis-à-vis des fibres, et
- un résidu réactif vis-à-vis des fibres de la série des halogénotriazines ou des halogénopyrimidines.

13. Utilisation des colorants réactifs selon une des revendications 1 à 9 ou des mélanges de colorants selon la revendication 10 pour la teinture ou l'impression de matériaux fibreux contenant des groupes hydroxyle ou des atomes d'azote.

14. Utilisation selon la revendication 13 pour la teinture ou l'impression de matériaux fibreux contenant de la cellulose ou de matériaux fibreux en polyamide naturel ou synthétique.
